# EUROPEAN PATENT SPECIFICATION

(11) **EP 2 452 948 B1**
(45) Date of publication and mention of the grant of the patent: **06.03.2019**
(21) Application number: 10796746.5
(22) Date of filing: 11.08.2010
(51) Int. Cl.: A61K 39/00, C07K 14/47, C07K 16/30, C07K 14/435, G01N 33/574, C07K 16/18, A61K 39/395, A61P 35/04

(54) **NEW TUMOR MARKER**
NEUER TUMORMARKER
NOUVEAU MARQUEUR TUMORAL

(30) Priority: 07.07.2009 CN 200910158747
(43) Date of publication of application: 16.05.2012
(73) Proprietor: Tsinghua University, Haidian District Beijing 100084 (CN); Protgen Ltd., Haidian District, Beijing 100085 (CN)
(72) Inventor: LUO, Yongzhang, Beijing 100084 (CN); SONG, Xiaomin, Beijing 100084 (CN); WANG, Xiaofeng, Beijing 100084 (CN); ZHUO, Wei, Beijing 100084 (CN); CHANG, Guodong, Beijing 100085 (CN); FU, Yan, Beijing 100084 (CN)
(74) Representative: Hoffmann Eitle
(86) International application number: PCT/CN2010/075896
(87) International publication number: WO 2011/003369

(56) References cited:
- WO-A2-00/68693
- WO-A2-2008/070472
- CN-A- 1 860 132
- LEES MILLER ET AL: "Two human 90-kDa heat shock proteins are phosphorylated in vivo at conserved serines that are phosphorylated in vitro by casein kinase II.", JOURNAL OF BIOLOGICAL CHEMISTRY, vol. 264, no. 5, 1 February 1989 (1989-02-01), pages 2431-2437, XP055056061, ISSN: 0021-9258
- K. SIDERA ET AL: "A Critical Role for HSP90 in Cancer Cell Invasion Involves Interaction with the Extracellular Domain of HER-2", JOURNAL OF BIOLOGICAL CHEMISTRY, vol. 283, no. 4, 1 January 2007 (2007-01-01), pages 2031-2041, XP055010921, ISSN: 0021-9258, DOI: 10.1074/jbc.M701803200
- MEHDI MOLLAPOUR ET AL: "Post-translational modifications of Hsp90 and their contributions to chaperone regulation", BIOCHIMICA ET BIOPHYSICA ACTA (BBA) - MOLECULAR CELL RESEARCH, vol. 1823, no. 3, 1 March 2012 (2012-03-01), pages 648-655, XP055056055, ISSN: 0167-4889, DOI: 10.1016/j.bbamcr.2011.07.018
- WANQ X.F. ET AL.: 'The regulatory mechanism of Hsp90alpha secretion and its function in tumor malignancy' PROCEEDINGS OF THE NATIONAL ACADEMY OF SCIENCES OF THE UNITED STATES OF AMERICA vol. 106, no. 50, 24 November 2009, pages 21288 - 21293, XP008148353
- BANERJI, U.: 'Heat Shock Protein 90 as a Drug Target: Some Like It Hot' CLINICAL CANCER RESEARCH vol. 15, no. 1, 01 January 2009, pages 9 - 14, XP008148356
- ZEKE, T. ET AL.: 'Human protein phosphatase 5 dissociates from heat-shock proteins and is proteolytically activated in response to arachidonic acid and the microtubule-depolymerizing drug nocodazole' BIOCHEMICAL JOURNAL vol. 385, no. 1, 01 January 2005, pages 45 - 56, XP008148351
- MANI S ET AL: "CLINICAL STUDIES IN PATIENTS WITH SOLID TUMORS USING A SECOND-GENERATION ANTISENSE OLIGONUCLEOTIDE (GEM 231) TARGETED AGAINST PROTEIN KINASE A TYPE I", ANNALS OF THE NEW YORK ACADEMY OF SCIE, NEW YORK ACADEMY OF SCIENCES, US, vol. 1002, 1 December 2003 (2003-12-01), pages 252-262, XP009051695, ISSN: 0077-8923, DOI: 10.1196/ANNALS.1281.028
- Y. S. CHO-CHUNG: "Tumor Reversion: Protein Kinase A Isozyme Switching", ANNALS OF THE NEW YORK ACADEMY OF SCIENCES, vol. 1058, no. 1, 1 November 2005 (2005-11-01), pages 76-86, XP055466224, US ISSN: 0077-8923, DOI: 10.1196/annals.1359.014

## Description

### Field of Invention

This invention relates to the diagnosis and therapy of tumor by analysis of samples, and specifically to a new tumor biomarker and methods and kits for the detection of cancer occurrence and metastasis. The invention also relates to methods of analyzing samples and medicaments for the treatment of cancer and/or its metastasis.

### Background of Invention

Currently, around 11 million people are diagnosed as tumor patients every year worldwide, and it is speculated that this number will increase to more than 16 million by the year of 2020. In 2005, among the 58 million deaths, 7.6 million are caused by cancer (accounting for about 13%). This number is increasing, and it is expected that 9 and 11.4 million people will die of cancer by the year of 2015 and 2030 respectively. (World Health Organization, 2006)

Tumor markers are the substances whose quantities are decreased or elevated by gene mutation or expression variation during the process of cell carcinogenesis. Normally, tumor markers include antigens and other bio-active substances, which can be used for the early detection of cancers as well as the monitoring of disease progression and response to a treatment (ASCO, 1996). It generates huge benefit for the clinical treatment of cancers, especially when it can detect cancer before any obvious clinical phenomenon or when it can be used to monitor the patients' response to a certain treatment. At present, in order to better meet the clinical need, greater efforts on the development of tumor biomarkers are required.

The applications of most tumor biomarkers currently used in clinic are more or less restricted due to the not-so-good sensitivity and specificity. For example, the AFP level and ultra-sonic examinations are largely used for liver cancer detection. Although their sensitivities are not very high, they indeed prolong the survival rate of the patients by diagnosis of the high-risk people. Tumor antigen CA-125 has a higher sensitivity but lacks specificity. Similarly, blood tumor biomarker CA15-3 for breast cancer could hardly be used for early detection due to its low sensitivity. Therefore, methods for cancer early detection and tumor malignancy determination are currently not available in clinic. Development of new technologies as well as new methods is required.

The development of tumor proteomics brings hope for the identification of new tumor biomarkers and for the screening, early diagnosis and prognosis of tumors. The malignant transformation of tumors always results in the change of protein expressions, which could be identified and quantitatively detected at the protein level. Thus, a lot of information and data could be derived, by which potential biomarkers could be discovered and evaluated for further development and clinical application.

Hsp90α (Heat shock protein 90α, Hsp90α) is a molecular chaperone, which functions to stabilize its client proteins in their active states. Hsp90α is one of the most abundant proteins in eukaryotic cells accounting for 1-2% of whole cell proteins. The intracellular Hsp90α mainly functions to stabilize its clients (i.e. estrogen receptor) and assistant their maturation (i.e. some kinases and signal proteins). Moreover, Hsp90α is also involved in other physiological events such as the evolution of mutated proteins, rearrangement of cytoskeleton, translocation of nuclear proteins, cell proliferation and apoptosis, protein degradation, antigen processing and LPS recognition etc. Hsp90α is also related with many diseases such as cancer, autoimmune disorder and cardiovascular diseases. For example, the monoclonal antibody against the antigen of LKVIRK sequence derived from Hsp90α can be used to treat fungal-related infection, and this clinical trial is currently ongoing by Neutec company (Trade name: Mycogrip).

It was also reported that Hsp90α could be secreted under some stimulus (Liao et al. (2000) J. Biol. Chem. 275, 189-96). Since Hsp90α is a classical intracellular protein, there is little report regarding the function of extracelluar Hsp90α. In previous literatures, Hsp90α was identified to facilitate the antigen processing in antigen processing cells (APCs) and was discovered to be one of the four proteins in lipopolysaccharide (LPS) activation clusters, which can interact with LPS thus trigger the response of intracellular proteins (Triantafilou et al. (2002) Trends in Immunology 23, 301-4).

Hsp90α was also found to be highly expressed in the surface of some tumor cells, including small-cell-lung cancer cells, melanoma and liver cancer cells (Ferrarini et al. (1992) Int. J. Cancer 51, 613-19). The high expression of cell surface Hsp90α in these cells were speculated to be related with the antigen processing while direct evidence is not available.

It was also reported that Hsp90α could help the translocation of transmembrane proteins (Schlatter et al. (2002) Biochem. J. 362, 675-84), and is related with the efflux of some drugs against leukemia, lung cancer, and cervix cancer (Rappa et al (2002) Oncol. Res. 12, 113-9 and Rappa et al (2000) Anticancer Drug Des 15,127-34).

The intracellular Hsp90α is an important target for anti-cancer drug development, as it is involved in the regulation of many signaling pathways which are critical for cell carcinogenesis. Inhibition of intracellular Hsp90α could result in the selective degradation of proteins related to cell proliferation, cell cycle control as well as apoptosis. Recently, some known antibiotics such as Geldanamycin, Radicicol and Coumermycin Al are discovered to be natural inhibitors of Hsp90α. A patent (WO 00/53169) describes this mechanism and proposes that prevention of the interaction between chaperones and clients is a method to inhibit chaperone activity. Among these antibiotics, coumarin and its derivatives are believed to have this activity. However, these inhibitors described in patent WO 00/53169 mainly target the intracellular Hsp90α.

The analogue of Geldanamycin 17-AAG is also an inhibitor of Hsp90α and is currently tested in clinical trials. However, some reports show that 17-AAG could have non-specific inhibitory effects and cell toxicity by interacting with many other cellular components. In addition, due to the limited knowledge on the varieties of cell signaling process involving Hsp90α and its clients, direct inhibition of intracellular Hsp90α is risky.

Another patent (EP1457499A1) describes the function of extracellular Hsp90α in facilitating tumor cell invasion *via* promoting the secretion or activation of the MMP-2. Based on these mechanisms, the patent proposes that inhibition of extracellular Hsp90α could prevent tumor invasion and metastasis, and by detecting the response of tumor cells to the treatment of Hsp90α inhibitor they can deduce the invasive ability of the cells and their relevance with Hsp90α.

The inventors of patent WO/2008/070472 propose that they can monitor the anti-tumor efficacy of Hsp90α targeted therapy by detecting the plasma Hsp90α and other related factors. In this patent, they provide the relevance between the plasma Hsp90α level and the efficacy of the Hsp90α targeted therapy using its inhibitors including 17-AAG and 17-DMAG, as well as the relevance between the level of plasma Hsp90α and tumor volume in mouse models. However, they did not provide any evidence about the exact form of plasma Hsp90α and did not demonstrate the relationship between the plasma Hsp90α level and tumor malignancy especially tumor metastasis. They did not propose the application of plasma Hsp90α as an independent tumor biomarker in tumor diagnosis and prognosis, either.

One group reported that serum Hsp90α level is related with the stages of non-small-cell lung cancer (Xu et al. (2007) J. Cancer Mol. 3,107-112). The serum level of Hsp90α in these lung cancer patients was significantly higher than that of normal people or benign tumor patients. However, again this paper did not identify the exact form of serum Hsp90α as well as its relationship with tumor metastasis. Besides, it only investigated non-small-cell lung cancer, the relevance and specificity between serum Hsp90α level and breast cancer, liver cancer, and pancreatic cancer are unknown. Moreover, this article only disclosed the qualitative change of serum Hsp90α in tumor patients, no information was available regarding the exact content of serum level of Hsp90α, and the cut-off value for normal or abnormal level which is necessary in tumor diagnosis and prognosis was not defined either.

### Summary of Invention

This invention is based on the discovery that the Hsp90α level in the blood is correlated with the development, malignancy and metastasis of many types of cancer. Accordingly, Hsp90α in the blood can be used as a new tumor biomarker. The inventors found that the Hsp90α in the blood is different from the intracellular Hsp90α in that it lacks four amino acid residues at its C-terminus compared with the intracellular Hsp90α.

Therefore, in one aspect, this invention provides an isolated polypeptide comprising or consisting of the amino acid sequence of SEQ ID No.1.

The polypeptide of this invention may be phosphorylated. In particular, in the polypeptide of this invention, one or more amino acid residues in the amino acid sequence of SEQ ID No.1 at positions selected from the group consisting of Thr90, Ser231, Ser263, Tyr309 and a combination thereof are phosphorylated. Preferably, the Thr90 in the polypeptide of this invention is phosphorylated.

The polypeptide of this invention may serve as a tumor biomarker. Using an agent specifically binding to the polypeptide of this invention, it is possible to detect the level of this polypeptide in the blood, and thereby to determine the presence, the degree of malignancy, and the metastasis of cancers.

Accordingly, in another aspect, this invention relates to the use of an agent capable of specifically binding to the polypeptide of this invention in preparation of a kit for determining the presence, stage and/or metastasis of a cancer in a subject through detecting the plasma level of the polypeptide of this invention, for screening the samples for the presence of a cancer in a high-risk population through detecting the plasma level of the polypeptide of this invention, for determining by sample analysis the prognosis of a patient having a cancer through detecting the plasma level of the polypeptide of this invention, for determining by sample analysis the efficiency of a surgery, radiotherapy or chemotherapy treatment to a patient having a tumor through detecting the plasma level of the polypeptide of this invention.

Preferably, the agent capable of specifically binding to the polypeptide of this invention is an antibody specific for the polypeptide. Preferably, the antibody is a monoclonal antibody or an antigen binding fragment thereof, such as scFv, Fab, Fab' and F(ab')2. In one specific embodiment, the antibody is MAb E9 or D10 produced by the cell line deposited under CGMCC No. 2903 or 2904, respectively.

According to this invention, the antibody specifically binds to the polypeptide of the invention, and preferably specifically binds to the polypeptide of the invention present in plasma. Preferably, the antibody specifically binds to a phosphorylated form of the polypeptide of the invention, said phosphorylated form of the polypeptide contains one or more phosphorylated amino acid residues in the amino acid sequence of SEQ ID No.1 at positions selected from the group consisting of Thr90, Ser231, Ser263, Tyr309 and a combination thereof. Preferably, the antibody specifically binds to the polypeptide which is phosphorylated at Thr90.

In another aspect, this invention relates to an inhibitor of the polypeptide of the invention for use for preventing or treating cancer metastasis in a subject. In one embodiment of this aspect, the inhibitor is an antibody specific for the polypeptide of the invention. Preferably, this antibody is a humanized antibody or an antigen binding fragment thereof. In one embodiment, the antibody specifically binds to a phosphorylated form of the polypeptide of the invention, said phosphorylated form of the polypeptide contains one or more phosphorylated amino acid residues in the amino acid sequence of SEQ ID No.1 at positions selected from the group consisting of Thr90, Ser231, Ser263, Tyr309 and a combination thereof. In a preferred embodiment, the antibody specifically binds to the polypeptide which is phosphorylated at Thr90. In one specific embodiment, the antibody is MAb E9 or D10 produced by the cell line deposited under CGMCC No. 2903 or 2904, respectively.

In the above aspects of this invention, the cancer can be, for example, lung cancer, liver cancer, gastric cancer, gastrointestinal cancer, esophagus cancer, osteosarcoma, pancreatic cancer, lymphoma, colorectal cancer, breast cancer, prostate cancer, oral cancer, nasopharyngeal cancer, cervical cancer, ovarian cancer, leukemia, malignant melanoma, sarcoma, renal cell carcinoma and cholangiocarcinoma.

This invention also involves antibodies that can specifically bind to the polypeptide of the invention which is present in plasma. In one specific embodiment, the antibody is MAb E9 or D10 produced by the cell line deposited under CGMCC No. 2903 or 2904, respectively. Preferably, the antibody of the invention is a humanized antibody or an antigen binding fragment thereof. In one embodiment, the antibody specifically binds to a phosphorylated form of the polypeptide, said phosphorylated form of the polypeptide contains one or more phosphorylated amino acid residues in the amino acid sequence of SEQ ID No.1 at positions selected from the group consisting of Thr90, Ser231, Ser263, Tyr309 and a combination thereof. In a preferred embodiment, the antibody specifically binds to the polypeptide which is phosphorylated at Thr90.

In another aspect, this invention relates to an agent for use in method of inhibiting cancer invasiveness and metastasis, comprising the step of inhibiting the phosphorylation of intracellular Hsp90α in cancer cells. In one embodiment, this invention relates to the agent for use in a method of inhibiting cancer invasiveness and metastasis, comprising the step of inhibiting the phosphorylation of Hsp90α the agent is for use in the method which at Thr90 in cancer cells. In one specific embodiment of this aspect, the agent is for use in the method which comprises a step of overexpressing a nucleic acid molecule encoding protein phosphatase 5 (PP5) in cancer cells, e.g., by way of gene introduction.

Specifically the invention relates to the following embodiments:
1. A method for determining the existence of malignant tumors or tumor metastasis which comprises measuring the Thr90 phosphorylated Hsp90α level of plasma samples from suspected tumor patients or suspected tumor metastasis patients.
2. The method according to item 1, wherein the method uses an antibody specific for the Thr90 phosphorylated Hsp90α.
3. A method for screening high-risk populations for cancer, for predicting the prognosis of cancer patients, or for determining whether the treatment of surgery, radiotherapy or chemotherapy on cancer patients is effective, by detecting the level of Thr90 phosphorylated plasma Hsp90α in a sample.
4. The method of item 3, wherein said cancer is selected from the group consisting of lung cancer, liver cancer, gastric cancer, gastrointestinal cancer, esophagus cancer, osteosarcoma, pancreatic cancer, lymphoma, colorectal cancer, breast cancer, prostate cancer, oral cancer, nasopharyngeal cancer, cervical cancer, ovarian cancer, leukemia, malignant melanoma, sarcoma, renal cell carcinoma and cholangiocarcinoma.
5. An agent for use in a method of inhibiting cancer invasiveness and metastasis, wherein the agent inhibits the phosphorylation of intracellular Hsp90α at Thr90 in cancer cells.
6. The agent for use according to item 5, wherein the agent is a nucleic acid molecule encoding protein phosphatase 5 in tumor cells.
7. The agent for use according to item 6, wherein said use comprises overexpression of the nucleic acid molecule which is achieved by gene introduction.
8. The agent for use according to item 6, wherein the protein phosphatase 5 comprises the amino acid sequence of SEQ ID NO: 5.
9. The agent for use according to any one of items 5-8, wherein said cancer is selected from the group consisting of lung cancer, liver cancer, gastric cancer, gastrointestinal cancer, esophagus cancer, osteosarcoma, pancreatic cancer, lymphoma, colorectal cancer, breast cancer, prostate cancer, oral cancer, nasopharyngeal cancer, cervical cancer, ovarian cancer, leukemia, malignant melanoma, sarcoma, renal cell carcinoma and cholangiocarcinoma.
10. An isolated polypeptide comprising or consisting of the amino acid sequence of SEQ ID NO: 1, wherein the Thr90 in the amino acid sequence of SEQ ID NO: 1 is phosphorylated.

### Brief Description of the Drawings

Figure 1: The plasma Hsp90α level in tumor-bearing mice is significantly increased than in that in normal mice.
Figure 2: The plasma Hsp90α level in cancer patients is significantly increased compared with that in normal people.
Figure 3: The titer assay of the mouse monoclonal antibody of E9 and D10.
Figure 4: The standard curve of plasma Hsp90α using mouse monoclonal antibody E9 and rabbit polyclonal antibody S2 (sandwich ELISA).
Figure 5: Quantitative measurement of plasma Hsp90α level in lung, liver, pancreatic cancer patients as well as benign galactoma and myoma of uterus patients using sandwich ELISA:
   A: The plasma Hsp90α level of liver cancer patients is measured by sandwich ELISA. The plasma Hsp90α level in benign tumor patients ranges from 2-10 ng/ml, mostly 2-5 ng/ml, while the plasma Hsp90α level in 69% (20/29) liver cancer patients is above 50 ng/ml, the average of which has more than 10-fold increment compared with that of benign tumor patients, which is consistent with the result of Western blotting. This indicates that the plasma Hsp90α level is positively correlated with tumor malignancy.
   B: The plasma Hsp90α level of lung cancer patients is measured by sandwich ELISA. The plasma Hsp90α level in 64% (9/14) lung cancer patients is above 50 ng/ml, the average of which has more than 10-fold increment compared with that of benign tumor patients. This indicates the plasma Hsp90α level is positively correlated with tumor malignancy.
   C: The plasma Hsp90α level of breast cancer patients is measured by sandwich ELISA. Compared with benign tumor patients, the highest plasma Hsp90α level is increased by more than 5-fold. Overall, the average plasma Hsp90α level in breast cancer patients shows significantly increment when compared with that in benign tumor patients.
   D: The plasma Hsp90α level of pancreatic cancer patients is measured by sandwich ELISA. The plasma Hsp90α level in 100% (10/10) pancreatic cancer patients is above 50 ng/ml, the average of which has more than 10-fold increment compared with that of benign tumor patients. This indicates the plasma Hsp90α level is positively correlated with tumor malignancy.

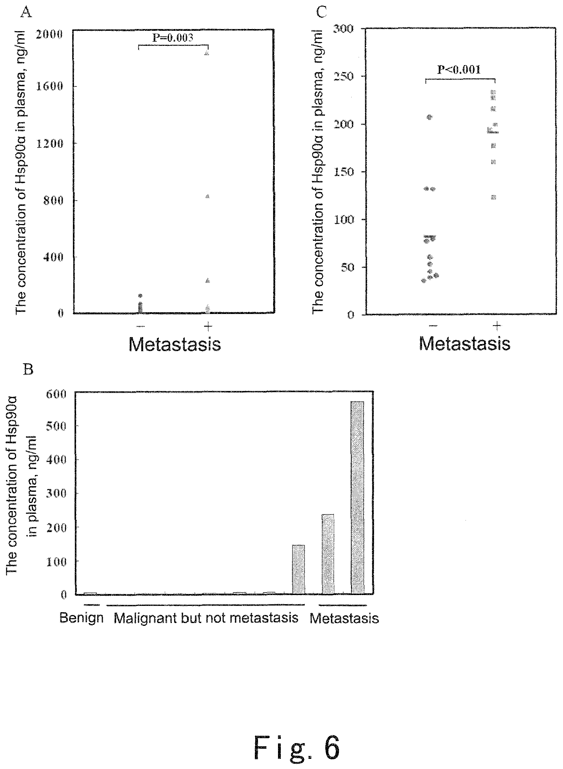

Figure 6: Quantitative measurement of plasma Hsp90α level in the cancer patients with or without metastasis using sandwich ELISA:
   A: The liver cancer patients are divided into two groups, one with metastasis and the other one without metastasis, then the plasma Hsp90α levels in these two groups are compared. The plasma Hsp90α levels in cancer patients with metastasis are all above 200 ng/ml while those in patients without metastasis range from 50 to 200 ng/ml.
   B: The lung cancer patients are divided into two groups, one with metastasis and the other one without metastasis, then the plasma Hsp90α levels in these two groups are compared. The plasma Hsp90α levels in cancer patients with metastasis are all above 200 ng/ml while those in patients without metastasis range from 50 to 200 ng/ml.
   C: The breast cancer patients are divided into two groups, one with metastasis and the other one without metastasis, then the plasma Hsp90α levels in these two groups are compared. The plasma Hsp90α levels in cancer patients with metastasis are significantly elevated compared with that in patients without metastasis.

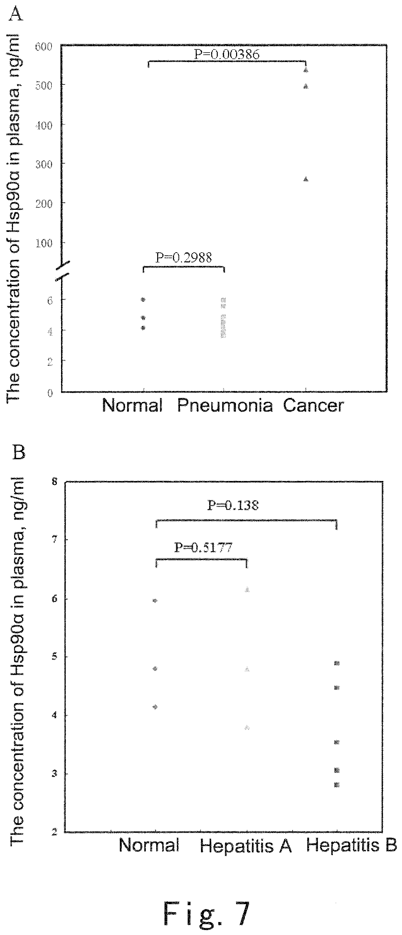

Figure 7: Quantitative measurement of plasma Hsp90α level in the patients with inflammation (pneumonia and hepatitis), normal people and tumor patients using sandwich ELISA:
   A: To ensure that the elevated plasma Hsp90α level of cancer patients is tumor specific, we compared the plasma Hsp90α level in pneumonia patients, normal people and tumor patients and found that the plasma Hsp90α level in pneumonia patients ranges from 2 to 10 ng/ml, indicating no significant changes compared with that in normal people.
   B: The plasma Hsp90α level in hepatitis patients (Hepatitis A and B) ranges from 2 to 10 ng/ml, indicating no significant changes compared with that in normal people.

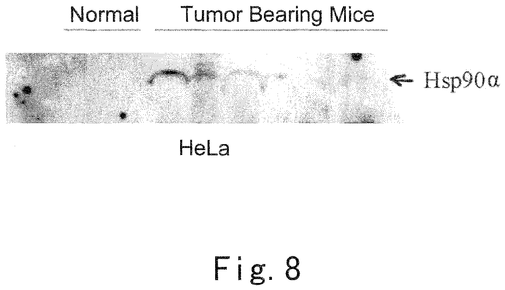

Figure 8: The plasma Hsp90α is secreted by tumor cells.

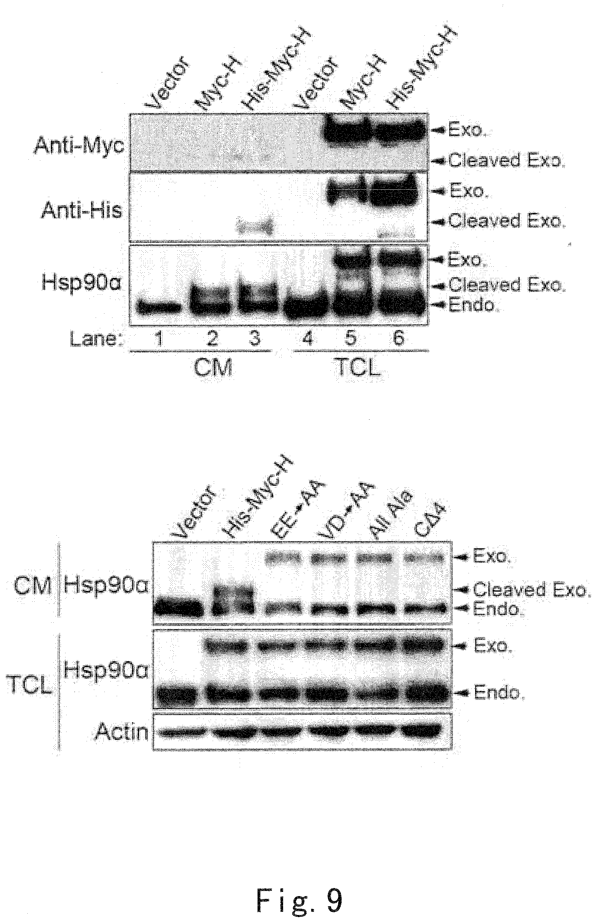

Figure 9: The secreted Hsp90α by tumor cells is in a C-terminal truncated form.

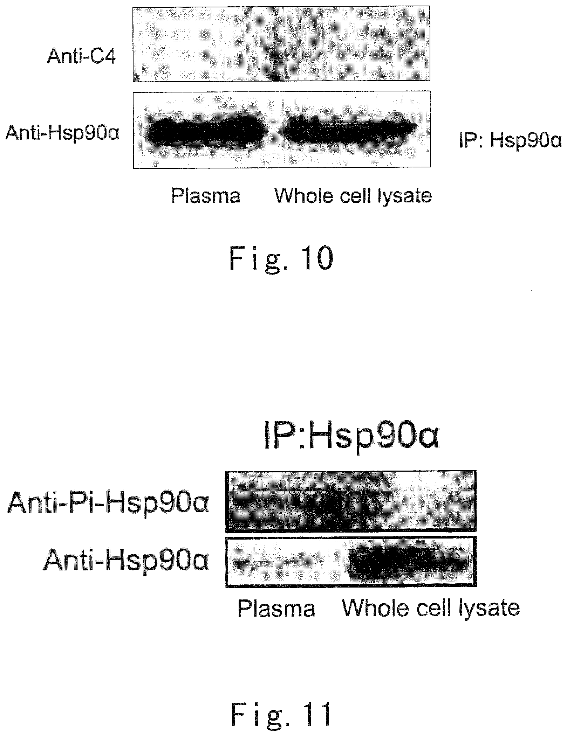

Figure 10: The plasma Hsp90α lacks the C-terminal four amino acid residues.
Figure 11: The plasma Hsp90α is phosphorylated.

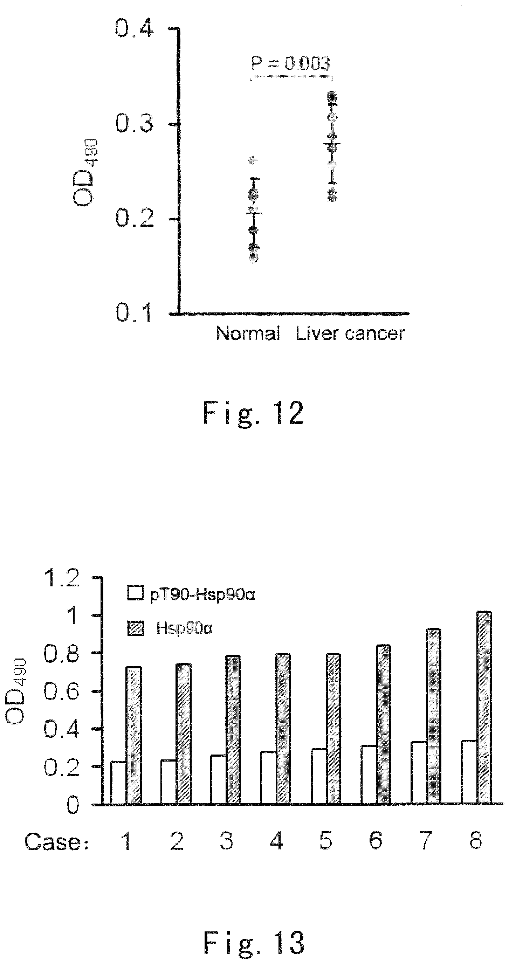

Figure 12: The Thr90 phosphorylated Hsp90α level in the tumor patient plasma is elevated.
Figure 13: In the plasma of tumor patient, the increment of Thr90 phosphorylated Hsp90α level is consistent with that of Hsp90α level.

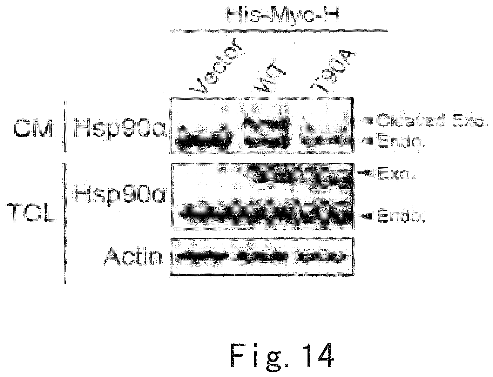

Figure 14: Thr90 Phosphorylation of Hsp90α is a pre-requisite for Hsp90α secretion.

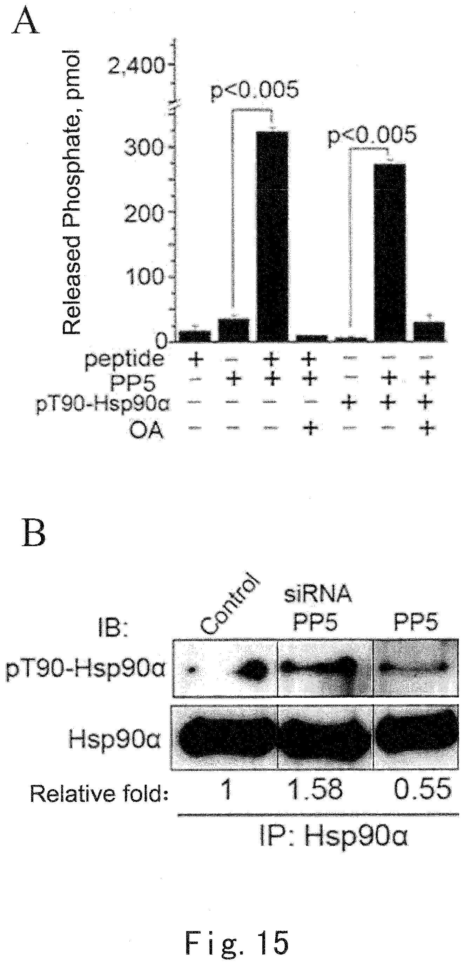

Figure 15: PP5 dephosphorylates Thr90 phosphorylated Hsp90α.
   A: Purified PP5 and Thr90 phosphorylated Hsp90α (pT90-Hsp90α) are incubated together, and the released free phosphate group is quantitatified. Peptide is a positive control. The result shows PP5 can directly dephosphorylate Thr90 phosphorylated Hsp90α.
   B: In the human breast cancer cell line MCF-7, overexpression of human PP5 results in the inhibition of Hsp90α Thr90 phosphorylation (0.55 of that in control group) and RNA interference of endogenous human PP5 results in the increase of Hsp90α Thr90 phosphorylation (1.58 of that in control group)

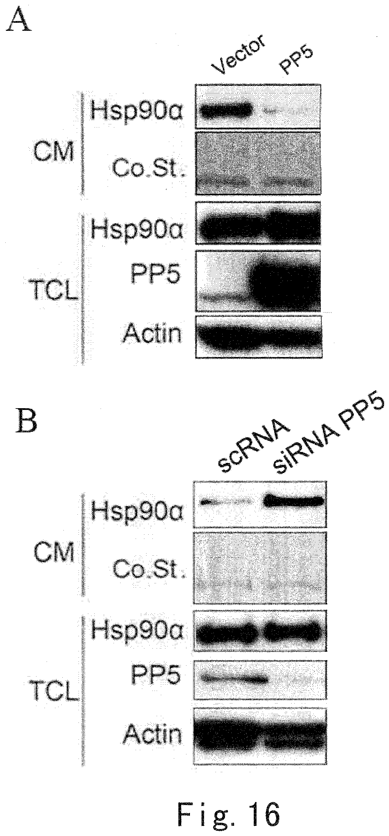

Figure 16: PP5 regulates the secretion of Hsp90α.
   A: In the human breast cancer cell line MCF-7, overexpression of human PP5 results in the inhibition of Hsp90α secretion.
   B: In the human breast cancer cell line MCF-7, RNA interference of endogenous human PP5 results in the increase of Hsp90α secretion.

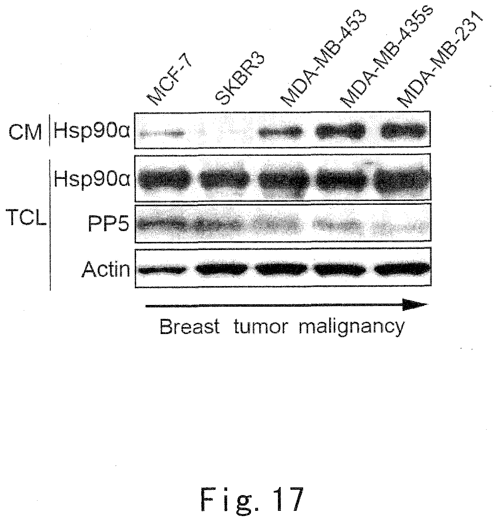

Figure 17: The correlation of PP5 expression level and the secretion level of Hsp90α.

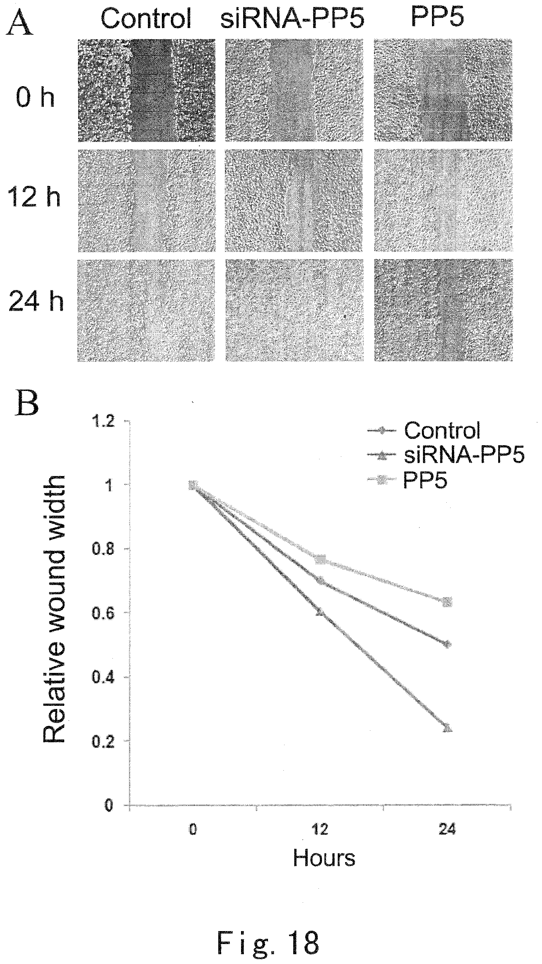

Figure 18: The correlation of PP5 expression level and the invasive ability of the tumor cells.

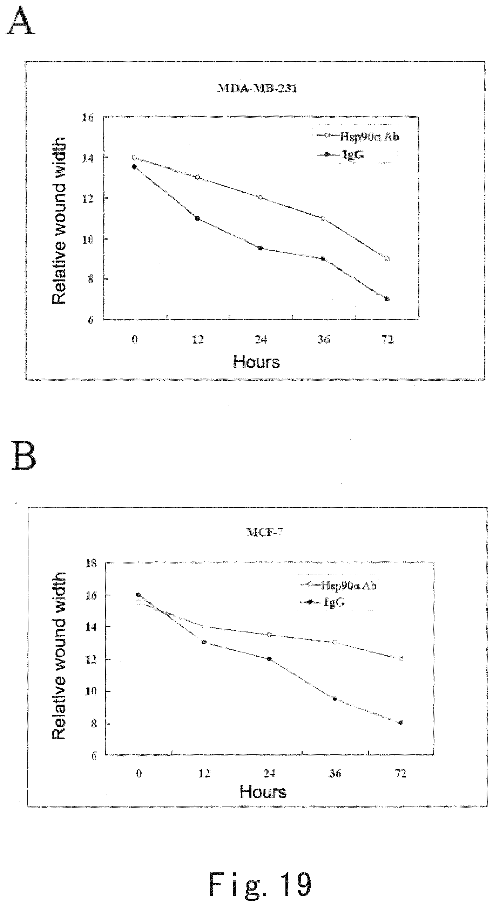

Figure 19: The specific antibody of Hsp90α could inhibit the migration of tumor cells.

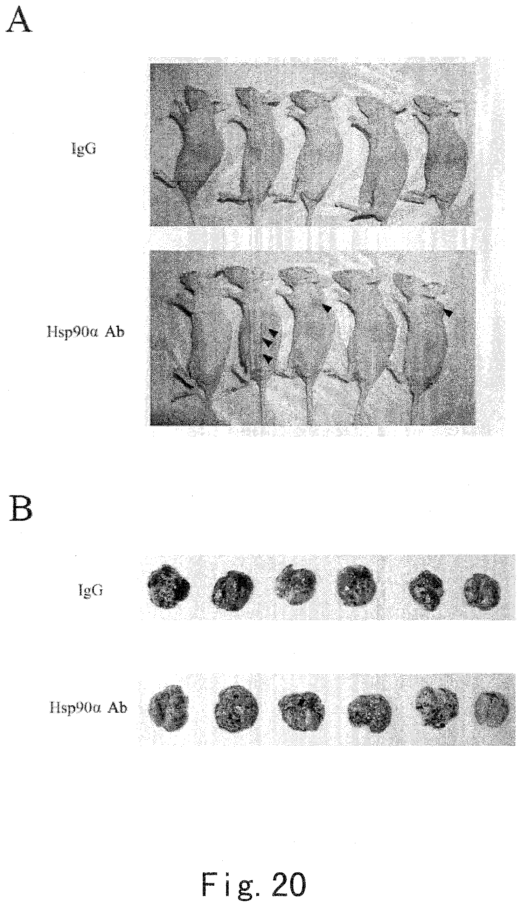

Figure 20: The specific antibody of Hsp90α could inhibit tumor metastasis.

### Information of Biological Material Deposition

Mouse hybridoma cell line SP2/0-Ag14 which produces monoclonal antibody E9 was deposited at China General Microbiological Culture Collection Center (CGMCC, Chinese Academy of Sciences Institute of Microbiology, Datun Road, Chaoyang District, Beijing) on February 24, 2009 with the Deposition No. CGMCC No.2903.

Mouse hybridoma cell line SP2/0-Ag14 which produces monoclonal antibody D10 was deposited at China General Microbiological Culture Collection Center (CGMCC, Chinese Academy of Sciences Institute of Microbiology, Datun Road, Chaoyang District, Beijing) on February 24, 2009 with the Deposition No. CGMCC No.2904.

### Detailed Description of the Invention

Carcinogenesis is caused by changes of certain intracellular signal transduction pathways, accompanied by changes of protein expression, modification and distribution. These changes could be used to monitor tumor development and progression, and these proteins are named as tumor markers. With the development of proteomics technology, it becomes possible to monitor the changes of tumor proteome qualitatively or quantitatively. So many new tumor markers have been found, providing more accurate and credible evidences for the tumor clinical diagnosis and prognosis.

This invention is based on a discovery of a new plasma tumor marker, i.e., plasma Hsp90α. Compared to intracellular Hsp90α (the amino acid sequence of which is SEQ ID No.3, and the coding nucleic acid sequence is SEQ ID No.4), the Hsp90α in plasma has a deletion of 4 amino acids in the C terminus.

Accordingly, in one aspect, this invention provides an isolated polypeptide, the Hsp90α found in plasma or serum which comprises or consists of the amino acid sequence of SEQ ID No.1. The term "polypeptide of the invention" used herein refers to the Hsp90α present in serum or plasma, which comprises or consists of the amino acid sequence of SEQ ID No.1. Preferably, the term "polypeptide of the invention" used herein refers to the polypeptide consisting of the amino acid sequence of SEQ ID No.1 sequence. In this application, the term "Hsp90α in plasma" or "Hsp90α in serum" can be used equally to refer to the free form of the protein Hsp90α present in the blood instead of intracellular or cell surface Hsp90α. In this application, term "polypeptide" and "protein" can be used interchangeably.

The present invention also provides a polynucleotide encoding a polypeptide composing or consisting of the amino acid sequence of SEQ ID No.1. In a specific embodiment, the polynucleotide comprises or consists of the amino acid sequence of SEQ ID No.2.

The inventors also found that the polypeptide of the invention is in a phosphorylated form in plasma in which one or more amino acid residues in the amino acid sequence corresponding to SEQ ID No.1 selected from the group consisting of Thr90, Ser231, Ser263, Tyr309 and a combination thereof are phosphorylated. Preferably, the Thr90 in the polypeptide of this invention is phosphorylated.

The special form of plasma Hsp90α (C-terminal truncated and phosphorylated) has not been described. Meanwhile, plasma Hsp90α also has never been reported to be related to tumor development and progression. EP1457499A1 described the extracellular form of Hsp90α, and suggested that the inhibitors of Hsp90α can be used to treat tumor metastasis, to detect tumor invasiveness and to determine the dependence of tumor invasiveness on Hsp90α. However, the method described in EP1457499A1 is used to detect cell surface Hsp90α, and it failed to mention plasma Hsp90α. Nor did EP1457499A1 disclose or suggest that plasma Hsp90α can be used to determine the degree and stage of malignancy, or to monitor the therapeutic response and prognosis of cancer by measuring the level of Hsp90α. WO/2008/070472 reported that the effects of the anti-cancer treatment targeting Hsp90α could be determined through detecting Hsp90α and its related factors in plasma, but it did not mention Hsp90α as an independent tumor marker in cancer diagnosis and prognosis.

The inventors examined the blood samples from nearly a hundred tumor patients (with breast cancer, liver cancer, pancreatic cancer, lung cancer et al), and found that the level of Hsp90α in plasma is correlated to tumor malignancy, particularly metastasis; while the inflammatory response does not influence plasma level of Hsp90α. Therefore, plasma Hsp90α can serve as a tumor marker, which can be used in the diagnosis and prognosis of tumors and metastasis.

Accordingly, in another aspect, the disclosure provides a kit for examining the plasma levels of the polypeptide of the invention. The kit of the disclosure contains an agent capable of specifically binding to the polypeptide of the invention. The kit of the invention can be used to detect the level of plasma Hsp90α.

The disclosure also relates to the use of an agent capable of specifically binding to the polypeptide of the invention in manufacturing a kit for detecting the level of plasma Hsp90α in a plasma sample of a subject. The kit of the disclosure can be used to determine the presence, malignancy and metastasis of tumor; to screen cancer in high-risk population; to determine the prognosis of cancer patients; and to determine the efficacy of surgery, radiotherapy or drug therapy and/or to determine whether or when a therapy should be ceased.

As used herein, the term "an agent capable of specifically binding to a polypeptide of the invention" refers to molecules which can bind to the polypeptide of this invention with high-affinity. These agents also include molecules which can bind intracellular and cell surface Hsp90α. An agent capable of specifically binding to the polypeptide of the invention can be proteins, especially Hsp90α specific antibodies. In the preferred examples, the above antibody is a monoclonal antibody or antigen binding fragment, such as scFv, Fab, Fab 'and F (ab') 2. In a specific embodiment, the antibodies are monoclonal antibody E9 or D10 which is produced by the cell line with deposition number of CGMCC No. 2903 or 2904, respectively.

Monoclonal antibodies are obtained by screening the cells which can secret the antibodies and then culturing the cells in vitro. The method is well known by a skilled person in this field (Köhler G & Milstein C. (1975) Nature. 256, 495-7). The process for preparing an Hsp90α-specific monoclonal antibody is as follows: for the first immunization, recombinant human Hsp90α (rhHsp90α, 100 µg) with Freund's complete adjuvant was injected subcutaneously on the back of BALB/c mice multi-pointly; 3 weeks later, same dose of rhHsp90α was injected intraperitoneally (i.p.) with Freund's incomplete adjuvant; the third immunization was administered by i.p. injection after 3 weeks with same dose (5 to 7 days later the blood titer was tested); after another 3 weeks, 200 µg rhHsp9aα was administered for the booster immunization by intraperitoneal injection. 3 days later, spleen cells were fused with hybridoma SP2/0-Ag14 (SP2/0) (Source: ATCC CRL-1581) using HAT for screening. Then hybridoma cells were limited diluted. Immune blot and ELISA methods were used to identify and eventually to select cell lines that can secrete specific Hsp90α antibodies.

According to the disclosure, the antibodies used for the preparation of the kit can specifically bind to Hsp90α, and preferably to Hsp90α in plasma. In one embodiment, the antibodies specifically bind to Hsp90α in which one or more following amino acid residues are phosphorylated: Thr90, Ser231, Ser263, Tyr309 and the combination thereof. In a preferred embodiment, antibodies described herein can specifically bind to Hsp90α with phosphorylated Thr90.

The invention also provides a method for detecting the plasma level of the polypeptide of the invention. The level of plasma Hsp90α can be detected by any suitable methods. The methods described herein include both direct and indirect means for detecting the polypeptide of the invention which can be used for the diagnosis of tumor development, malignancy and metastasis.

Direct measurements include methods of detecting the described polypeptide of the invention using an agent capable of specifically binding to said polypeptide, for example using specific antibodies of the polypeptides by Western blotting or ELISA.

The concentration of Hsp90α can also be indirectly determined by detecting the activity of Hsp90α. An example is the assay of thermal induced denaturation of luciferase, which can be used to detect the chaperone activity of Hsp90α (Johnson et al. (2000) J. Biol. Chem., 275, 32499-32507).

Preferably, the level of plasma Hsp90α can be detected by ELISA or Western blotting, comprising the steps as follows:
a) collecting the whole blood of a subject, such as a cancer patient, and obtaining plasma or serum by centrifugation;
b) using ELISA or Western blotting to detect the Hsp90α level of plasma or serum obtained from the step a), in which plasma sample of healthy people is used as the negative control, and plasma sample of patients with malignant tumors is used as the positive control, and optionally generating an Hsp90α concentration standard curve; and
c) determining the tumor malignancy and stage according to the level of plasma Hsp90α, and thereby determining the diagnosis, prognosis or efficacy of treatment for the subject.

In step b), other methods can also be used, such as other methods based on antigen-antibody reactions and methods based on other principles directly or indirectly reflecting the concentrations of Hsp90α, for example, methods for determining the concentration of Hsp90α by detecting the activity of Hsp90α.

Standard Hsp90α sample used for generating the standard curve of ELISA can be purified from the plasma of cancer patients and can also be obtained by recombinant techniques. Standard Hsp90α sample can be the full length Hsp90α, its fragments, and other recombinant proteins or conjugates containing the sequence of the Hsp90α. "the standard curve of Hsp90α concentration" refers to the correlation curve between Hsp90α concentration and absorbance values detected in the ELISA using standard Hsp90α samples. "Standard Hsp90α sample" refers to the plasma Hsp90α protein, recombinant Hsp90α protein, the fragments or derivatives thereof with a purity of above 95%.

"Determining the malignancy of tumor" refers to making a judgment of tumor malignancy by examining the Hsp90α concentration in the plasma samples of patients and comparing this value with negative and positive controls.

Both sandwich and competitive ELISA can be used to detect the level of Hsp90α in plasma. The competitive ELISA with higher sensitivity is preferred.

Generally, Sandwich ELISA includes the steps of: a) immobilizing a specific antibody onto a solid support, and removing the unbound antibodies and impurities by one or more washing steps; b) adding samples to be tested and incubating for a period of time, allowing the antigen in the samples to bind to the immobilized antibody and form an antigen-antibody immune complex on the solid support, and then removing the unbound substances; c) adding an enzyme-conjugated-antibody which can bind to the antigen in the solid phase immune complexes, and then removing the unbound enzyme-conjugated-antibody by thorough washes (the amounts of enzyme-conjugated-antibody bound to the solid support are positively correlated to the amount of antigen to be tested); and d) adding substrate and qualitatively or quantitatively determining the amount of antigen according to the color reaction.

Generally, competitive ELISA includes the steps of: a) immobilizing a specific antibody onto a solid support and washing thoroughly; b) adding a mixture of the sample to be tested and a certain amount of enzyme-conjugated-antigen, and incubating for enough time and wash thoroughly. (If there is no antigen in the sample, the enzyme-conjugated-antigen will bind to the solid phase antibody without competition. If there is antigen in the sample, the antigen in the sample will compete with the enzyme-labeled-antigen to bind the immobilized antibody, and the amount of the enzyme-labeled-antigen bound to the immobilized antibody will be decreased. As a control, the reference tube only contains the enzyme-labeled antigen); c) adding substrate and obtaining the absorbance value for each tube. The value of the reference tube is highest, and the difference between the reference tube and the sample tube represents the amount of antigens in the sample. The lighter the color is, the more antigens the sample contains.

If Sandwich ELISA is used, the antibodies are two different species of plasma Hsp90α specific antibodies. The immobilized antibody can be a rabbit polyclonal antibody with strong binding capacities, while the detection antibody is a mouse monoclonal antibody with high specificity. These two antibodies should have no cross-reaction.

If competitive ELISA is used, the antibodies should be the specific antibodies against plasma Hsp90α, which should have strong binding capacities and high specificity to both the competitive substance and Hsp90α.

If competitive ELISA is used, the competitive substance can be a labeled plasma Hsp90α standard protein. And the label does not interfere with the binding of Hsp90α standard protein to its antibody.

If Western blotting method is used, the first antibody used is a specific antibody against plasma Hsp90α; the secondary antibody can be conjugated with horseradish peroxidase, or alkaline phosphotase; the substrate can be DAB or fluorescent substrate. The fluorescence substrate with high sensitivity is preferred.

The sensitivity of above methods should be 10 ng/ml or more sensitive.

The antibody specific for plasma Hsp90α used in this present invention can be a whole antibody, or its fragments and derivatives.

The antibodies of this invention can also be replaced by other agents capable of specifically binding to Hsp90α, wherein the agents include small molecule compounds, peptides and their derivatives.

The competitive plasma Hsp90α standard proteins can be labeled by biotin and various fluorescent labeling reagents. Biotin is preferred.

The cancers which can be detected by the kit or method described in this invention include, but are not limited to, lung cancer, liver cancer, gastric cancer, esophageal cancer, osteosarcoma, pancreatic cancer, lymphoma, colon cancer, breast cancer, prostate cancer, oral cancer, nasopharyngeal cancer, cervical cancer, leukemia, malignant melanoma, sarcoma, renal cancer, cholangiocarcinoma. "Tumor and its malignancy" refers to whether the tumor is benign, malignant, or metastatic.

The inventors demonstrated that, the plasma Hsp90α level in non-cancer human is 2-50 ng/ml, most in the range of 2-10 ng/ml. The level of plasma Hsp90α in patients diagnosed with cancer is higher than the normal level, while the plasma Hsp90α level in the patients having cancer metastasis is higher than 50 ng/ml, mostly higher than 200 ng/ml. This makes the plasma Hsp90α a new tumor marker, which would be helpful for the diagnosis of cancer, especially metastasis.

Thus, in one embodiment, the kit of the disclosure or method of this invention can be used to determine the existence of tumor, especially malignant tumors and tumor metastasis. To this end, the kit of the disclosure or method of this invention can be used to measure the Hsp90α level of plasma samples from suspected tumor patients or suspected tumor metastasis patients, and optionally compared with normal controls, and then determine the possibility of patients with tumor or tumor metastasis according to the Hsp90α level in the sample. The elevated Hsp90α level in plasma suggests higher possibility that the patients have suffered from malignant tumor, and for patients with known cancer, an elevated Hsp90α level strongly suggests possibility of tumor metastasis.

In another embodiment, the kit of the disclosure or method of the invention can be used to screen high-risk populations for tumors by detecting the level of plasma Hsp90α. To this end, the kit of the disclosure or method of the invention can be used to detect the plasma Hsp90α level in samples from high-risk populations, and optionally the detected level can be compared with normal controls, and then one can determine the possibility of an individual within the population to have tumor according to the level of the sample. Elevated Hsp90α level indicates the higher possibility of the presence of a malignant tumor. It is well-known for the people in this field how to determine different high-risk groups depending on the type of cancer to be screened, individual factors such as age, family history, lifestyle, work environment, history of exposure to harmful compounds and so on. For example, patients having chronic hepatitis B or hepatitis C are at high risk of HCC.

In another embodiment, the kit of the disclosure or method of the invention can be used to predict the prognosis of cancer patients by detecting the level of plasma Hsp90α. To this end, the kit of the disclosure or method of this invention can be used to detect the plasma Hsp90α level in a sample from a cancer patient, and optionally the detected level can be compared with normal controls or the previous levels of plasma Hsp90α of the patient, and then one can determine the prognosis of the cancer patient according to the level of Hsp90α in plasma samples. The maintenance of Hsp90α in a high level or a further increase of Hsp90α level may relate to the poor prognosis. Therefore, the clinicians can be alerted to provide more closely observation to the patient, and if necessary, change the current treatment.

In another embodiment, the kit of the disclosure or method of the invention can be used to detect the plasma level of Hsp90α, which is thus used to determine whether the treatment such as the surgery, radiotherapy or chemotherapy on cancer patients is effective. To this end, the kit of the disclosure or method of this invention can be used to detect the plasma Hsp90α level in a sample from a cancer patient, and optionally the detected level can be compared with normal controls or the previous levels of plasma Hsp90α of the patient, and then one can determine whether the surgery, radiotherapy or chemotherapy on the cancer patient is effective and/or whether or when the treatment should be ceased.

A person skilled in the art will understand that, like all the other known tumor markers or screening and diagnostic methods based on such tumor markers, the method of the invention for determining the presence or metastasis of tumor by detecting the level of Hsp90α in a plasma sample is an auxiliary method useful in the procedure of tumor diagnosis. Generally, the results obtainable by the methods of the invention *per se* are not sufficient for one skilled in the art to directly make an immediate judgment whether a subject is suffering from a malignant tumor or whether there is metastasis, nor can it be directly used to determine the histological origin and pathological feature of the malignant tumor or the sites and number of metastasis. Therefore, the establishment of a clinical diagnosis still depends on the results of clinical imaging technique and pathological examination as well as the judgment of clinical oncologists. As such, the purpose of the methods of the invention is to provide clinical practitioners or researchers with some additional information for further reference. Nonetheless, as demonstrated herein, the level of plasma Hsp90α will provide useful information for determining the presence of malignant tumor, especially cancer metastasis. Likewise, the methods of the invention for tumor screening, prognosis evaluation, and therapeutic efficacy evaluation can also provide useful information for clinical practitioners or researchers.

The inventors further demonstrated that the Hsp90α in plasma is secreted by tumor cells and is different from that within the tumor cells. Therefore, it is assumed that the tumor development and metastasis may be suppressed by inhibiting the secretion of Hsp90α into plasma. This was demonstrated on a mouse tumor metastasis model using the specific antibody against plasma Hsp90α. Therefore, the secretion of Hsp90α can be used as a new target for screening new anticancer drugs.

In another aspect, the invention relates to the use of an inhibitor of the polypeptide of the invention for use in a method for the prevention or treatment of tumor metastasis.

According to one embodiment of this invention, the aforementioned inhibitor is a specific antibody of Hsp90α. Preferably, the antibody is a humanized antibody or a fragment thereof. In one embodiment, these antibodies specifically bind to a phosphorylated form of Hsp90α which contains one or more phosphorylated amino acid residues in the amino acid sequence corresponding to SEQ ID No.1 selected from the group consisting of Thr90, Ser231, Ser263, Tyr309 and a combination thereof. In a preferred embodiment, the antibody can bind to Hsp90α which is phosphorylated at Thr90. In one specific embodiment, the antibody is MAb E9 or D10 which is produced by the cell line deposited under CGMCC No. 2903 or 2904, respectively. As demonstrated herein, these antibodies can completely inhibit the lymph node metastasis of tumors in mouse model and can inhibit the lung metastasis to an extent up to 56%.

Accordingly, in another aspect, this invention also relates to an antibody that specifically binds to the polypeptide of the invention, and in particular the Hsp90α in plasma. In a specific embodiment, the antibody is MAb E9 or D10 which is produced by the cell line deposited under CGMCC No. 2903 or 2904, respectively. Preferably, the antibody is a humanized antibody or an antigen binding fragment thereof. In a specific embodiment, the antibody specifically binds to a phosphorylated form of Hsp90α which contains one or more phosphorylated amino acid residues selected from the group consisting of Thr90, Ser231, Ser263, Tyr309 and a combination thereof. In one preferred embodiment, the antibody specifically binds to the Hsp90α phosphorylated at Thr90. Preferably, the antibody inhibits tumor growth, especially metastasis. This invention also related to a conjugate comprising the antibody and a diagnosis or treatment moiety. For example, the diagnosis moiety is a fluorophore, while the treatment moiety is a chemotherapeutic agent.

The inventor also found the amount of Hsp90α secreted by tumor cells is related to the level of Protein phosphatase 5 (PP5) in tumor cells. In benign tumor, secreted Hsp90α is low but PP5 expression level is high. In malignant tumor, secreted Hsp90α is high but PP5 expression level is low. Therefore, the level of secreted Hsp90α is negatively interrelated with the level of PP5. As a result, by detecting the expression level of PP5 in a tumor tissue sample, it is possible to predict the amount of secreted Hsp90α.

The inventors also found that the secretion of Hsp90α can be inhibited by intracellular PP5. Over-expression of a nucleic acid molecule encoding PP5 in the cell can inhibit Hsp90α secretion, while a decrease in the expression level of PP5 will result in an increase in Hsp90α secretion. So by over expressing PP5, Hsp90α secretion can be inhibited and the tumor progression and metastasis may be inhibited. Based on our experiment, we found that over expression of PP5 inhibits the migration of MCF-7 breast cancer cells. So PP5 can be a new therapeutic target for tumor treatment.

Accordingly, in a further aspect, this invention provides an agent for use in a method for inhibiting tumor invasiveness and metastasis, comprising a step of inhibiting the phosphorylation of Hsp90α within tumor cells. In one embodiment, the agent is for use in the method which comprises a step of inhibiting Thr90 phosphorylation of Hsp90α in tumor cells. In one specific embodiment, this method comprises a step of over-expressing a nucleic acid molecule encoding PP5 in tumor cells. Preferably, the over-expression of PP5 is achieved by the means of gene introduction. In one embodiment, this method comprises a step of over-expressing a nucleic acid molecule encoding PP5 having an amino acid sequence of SEQ ID No.5. In one specific embodiment, the nucleic acid molecule comprises the nucleotide sequence of SEQ ID No.6. This invention also relates to the use of a vector carrying a polynucleotide encoding PP5 operably linked to a promoter in preparation of a medicament for inhibiting the phosphorylation of Hsp90α in tumor cells by over-expressing PP5 in tumor cells. The medicament can be used to inhibit tumor invasiveness and metastasis.

This invention also provides methods and models for screening for anti-tumor drugs by using plasma Hsp90α or its derivatives. Such anti-tumor drugs include, but not limited to, plasma Hsp90α binding proteins, small peptides, and small compounds as well as inhibitors which can suppress the activity of plasma Hsp90α.

### Examples

### Example 1: The collection and preparation of mouse plasma samples, and the detection of plasma Hsp90α.

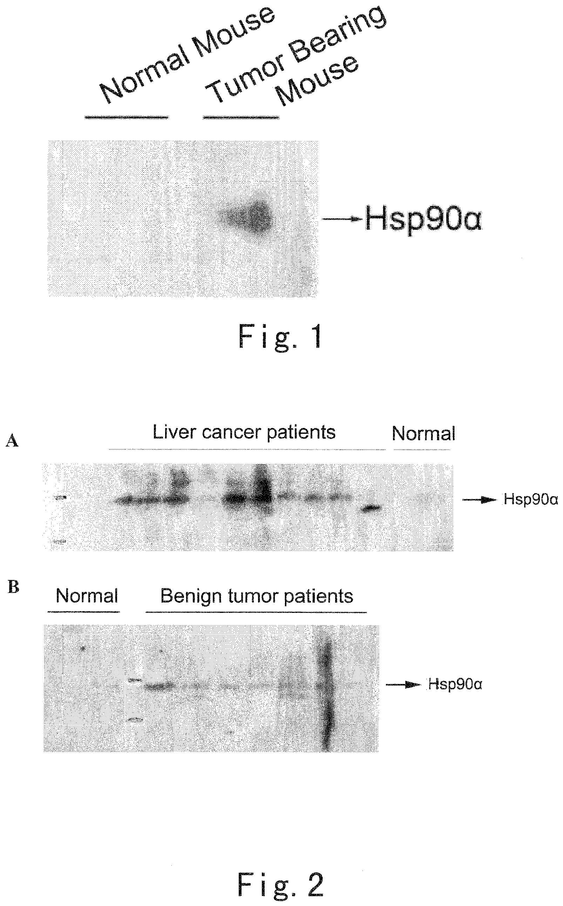

Balb/c mice with average body weight of 20 gram (purchased from Beijing Vital River Laboratory Animal Technology Co., Ltd.) were randomized divided into two groups, 3 for each. The mice of experiment group were inoculated with 10⁶ H22 (CCTCC ID: GDC091) cells, while the mice in control group were not inoculated. When the diameter of tumor reached about 2 cm (about 20 days), blood was collected from eye down veniplex. Anticoagulant was added and hemolysis was prevented. If hemolysis occurred, the blood would be recollected. The blood sample was centrifuged at 4°C with 6000g for twice, and supernatant was saved. The amount of plasma Hsp90α was detected by Western blotting using Rabbit anti-human Hsp90α pAb (Labvasion). BCA method (Pierce) was used to determine the total amount of proteins in samples, which ensured that the loading amounts of different samples were equal. The result is shown in Fig.1. Compared to control mice, the plasma Hsp90α was elevated in tumor bearing mice.

### Example 2: The collection and preparation of plasma samples from normal people and tumor patients, and the detection of plasma Hsp90α.

The blood of normal people and cancer patients were collected and delivered to lab within 24 hours at 4°C. If hemolysis occurred, the samples should be recollected. The samples were centrifuged at 4°C, 6000g twice and supernatant was saved. The amount of Hsp90α was determined by Western blotting. If the samples could not be examined immediately, the samples should be stored at -80°C. By comparing the results with clinical diagnosis, the correlation between Hsp90α and tumor malignancy was confirmed.

The protocol for Western blotting: the plasma samples were mixed 1:1 with loading buffer. 1-2 µl sample is loaded for SDS-PAGE. Primary antibody is the one which can specifically recognize plasma Hsp90α(rat mAb SPA-840, Stressgen). The secondary antibody is goat anti-rat antibody conjugated with HRP (purchased from Zhongshanjinqiao). As the result shows in Fig.2, the amount of Hsp90α in liver cancer patients is 10-fold higher than that of normal people (A), while it is elevated by 2-fold in benign galactocele and hysteromyoma patients compared with that of normal people.

### Example 3: Preparation of rabbit pAb and mouse mAb against Hsp90α

Primers with the following sequences were used to clone the gene of Hsp90α: Hsp90α-Sal1-Re: ACGCGTCGACTTAGTCTACTTCTTCCATGC (SEQ ID No.8) and Hsp90α-Sph1-For: ACATGCATGCATGCCTGAGGAAACCCAGACC (SEQ ID No.9). Primers were synthesized by Invitrogen. Pfu DNA polymerases (NEB) were used to amplify Hsp90α from human liver cDNA library (Stratagene). Sph1 and Sal1 (NEB) were used to digest the amplified PCR products and pQE80L vector (Qiagen). Then T4 ligases were used for ligation. The products were transformed into Top10 cells (Transgen) for amplification and validation. The validated plasmids were further transformed into BL21DE3 cells (Transgen) for expression. The recombinant Hsp90α proteins were purified by ion-exchange chromatography SP HP, pH6.8, collecting 10 ms/ml elution peak and Q HP, pH7.8, collecting 19 ms/ml elution peak.

Recombinant human Hsp90α with a purity higher than 95% was used to immune adult male New Zealand Rabbit by dorsal subcutaneous multi-point injection with a total amount of 100 µg. Two weeks later, the secondary immunization was conducted according to the same method, except that the amount of Hsp90α injection was reduced to 50 µg. Boost injections were conducted every 2 week after the secondary immunization for twice. The titer of antibody in serum was determined 7-10 days after each boost immunization. Eight days after the last immunization, serum was collected and stored at -20°C. Affinity chromatography conjugated with antigen was used to purify the antibody from the serum. Purified rabbit pAb was named as S2.

BALB/C mice were immunized by recombinant human Hsp90α. Primary immunization: 100 µg antigen and Freund's complete adjuvant was injected dorsal subcutaneously multipoint. The second immunization was conducted 3 weeks later with Freund's incomplete adjuvant using the same dose and *i.p.* injection. The third immunization was conducted another 3 weeks later without adjuvant using the same dose (blood was collected for test after 5-7 days). Three more weeks later, 200 µg antigen was injected *i.p.* as the boost immunization. 3 days later, spleen cells were collected and fused with SP2/0-Ag14(SP2/0) hybridomas (ATCC: CRL-1581). HAT was used for the screening. Limited dilution was used to colonize hybridomas. Western blotting and ELISA were used for identification. Finally, E9 and D10, which secrete specific antibody against Hsp90α, were obtained and banked as CGMCC No.2903 and 2904 on February 24, 2009.

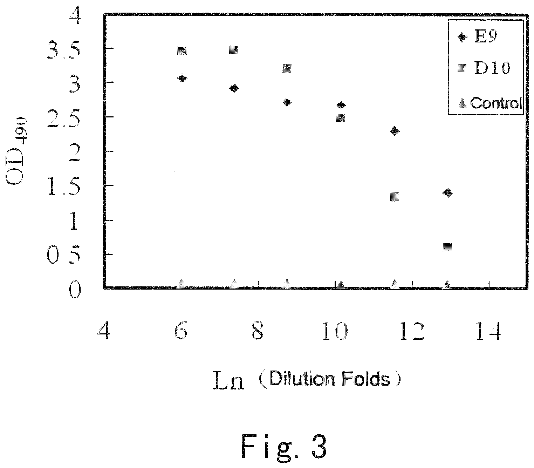

Indirect ELISA was used to determine the titers of E9 and D10. As shown in Fig.3, E9 and D10, with the titers of 500,000, can be used for the detection of plasma Hsp90α. Indirect ELISA: the plate was coated with recombinant human Hsp90α with the concentration of 10 µg/ml at 4°C overnight. Then the plate was blocked at 37°C for 1 hour. E9 or D10 with 1:400, 1:1600, 1:6400, 1:25600, 1:102400, 1:509600 dilutions was added and incubated for 2 hours at room temperature. Then the secondary antibody, goat anti-mouse antibody conjugated with HRP (purchased from Zhongshanjinqiao), was added and incubated for 1 hour at room temperature. O-phenylendiamine was added as the substrate and absorbance at OD 490 nm was detected.

### Example 4: Measurement of the concentration of Hsp90α by E9, S2 sandwich ELISA

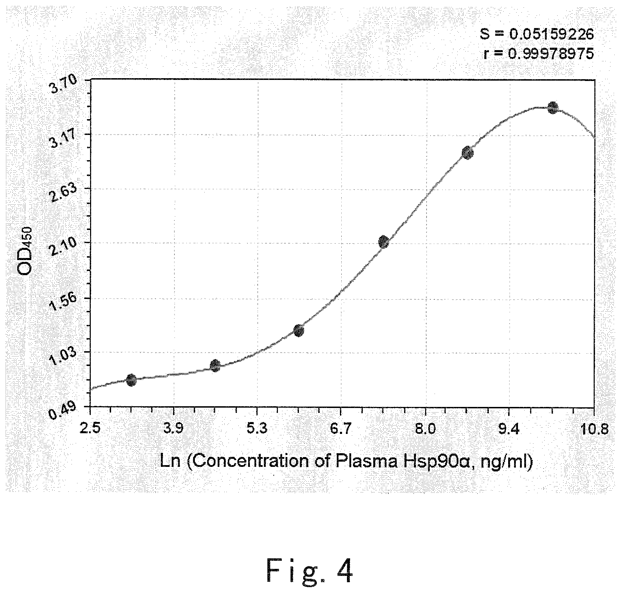

In the method of sandwich ELISA to test the concentration of plasma Hsp90α, two antibodies from distinct species were used. S2 with high binding capacity (the preparation of S2 was described in example 3) was used as the coating antibody and E9 (the preparation of E9 was described in example 3) with high binding specificity was used as the detecting antibody. There is no cross reaction between these two antibodies. The method is repeatable and sensitive. Fig.4 shows that the sensitivity of this method is 5 ng/ml.

### Example 5: The collection and preparation of human plasma, the detection of plasma Hsp90α and determination of tumor malignancy (sandwich ELISA).

The blood samples of healthy people, cancer patients and inflammation patients were delivered to lab at low temperature within 24 hours. If hemolysis occurrs, the blood should be re-collected. The samples were centrifuged at 4°C, 6000g twice and supernatant was saved. The amount of plasma Hsp90α was determined by ELISA. The samples were aliquoted and stored at -80°C if they were not examined immediately. By comparing the results with clinical diagnosis, the correlation between Hsp90α and tumor malignancy was confirmed.

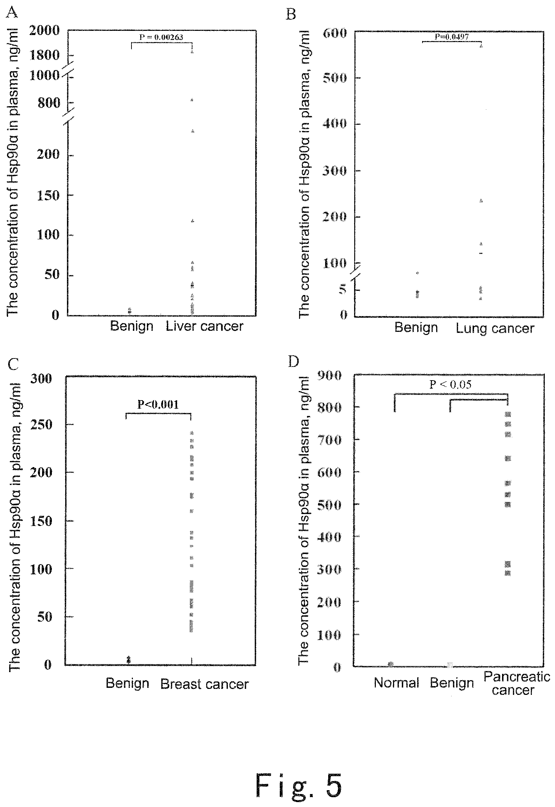

Two different Hsp90α antibodies were used in sandwich ELISA. S2 was coated on the plate and incubated overnight at 4°C. The plate was blocked for 1 hour at 37°C. The plasma samples were diluted by 10 folds and were added to the plate (100 µl/well). After 2 hours incubation at 37°C, E9 was added and incubated at 37°C for 2 more hours. Goat anti-mouse antibody conjugated with HRP was added to incubate for another 1 h. O-phenylendiamine was added and absorbance at OD490 nm was detected. The results were shown as Fig.5, 6 and 7. The standard curve comprises a serial of samples containing the gradient concentration of standard Hsp90α proteins and 10% of negative plasma in each sample, which was used to exclude the background of plasma.

As shown in Fig.5, the amount of plasma Hsp90α in benign tumor patients (including benign galactocele and hysterromyoma patients, seven cases) is between 2-10 ng/ml, mainly in 2-5 ng/ml. The level of plasma Hsp90α in 69% (20/29) of liver cancer patients is above 50 ng/ml, which is in average 10 folds higher than that of benign tumor patients, p=0.00263, student t-test (A); The level of plasma Hsp90α in 64% (9/14) lung cancer patients is above 50 ng/ml. The average concentration is 10 folds higher than that of benign tumor patients, p=0.0497, student t-test (B). The level of plasma Hsp90α in 78% (25/32) breast cancer patients is above 50 ng/ml. The average is 10 folds higher than that of benign tumor patients, p<0.001, student t-test (C). The level of plasma Hsp90α in 100% (10/10) pancreatic cancer patients is above 50 ng/ml. The average is 10 folds higher than that of benign tumor patients, p<0.05, student t-test (D).

As shown in Fig.6, in liver cancer (17 cases, 7 cases with metastasis) (A), lung cancer (10 cases, 2 cases with metastasis) (B) and breast cancer (21 cases, 10 cases with metastasis) (C) patients, the levels of Hsp90α in metastasis patients were significantly higher than those in patients without metastasis. Liver cancer p=0.003, breast cancer p=0.002, student t-test.

As shown in Fig.7, inflammation patients, including 10 pneumonia cases (A), 10 hepatitis cases (type A 5 cases, type B 5 cases) (B) have plasma Hsp90α between 2-10 ng/ml, which shows no significant difference when compared with that of normal persons (3 cases), p=0.2988, 0.5177, 0.138 by student t-test, respectively.

The normal people's samples were collected from healthy volunteers, while tumor patients and inflammation patients' samples were collected from Beijing Cancer Hospital and Xiamen First Hospital.

### Example 6: The plasma Hsp90α was secreted by tumor cells

Nude mice (purchased from Beijing Vital River Laboratory Animal Technology Co., Ltd.) with average body weight of 20 g were divided into two groups, 6 mice for each group. Each mouse was injected with 10⁶ Hela cells (ATCC: CCL-2). Control group was normal mice without tumor. When the diameter of tumor reached 2 cm (about 20 days), blood was collected from eye veniplex. Hsp90α antibody (rat mAb, Stressgen) that specifically recognizes human Hsp90α but not mouse Hsp90α was used to detect the plasma Hsp90α. As shown in Fig.8, the plasma Hsp90α in the tumor-bearing mice is specifically recognized by human but not mouse Hsp90α antibody, which indicates that the plasma Hsp90α was secreted by the xenograft tumor cells.

### Example 7: Hsp90α secreted by tumor cells was C-terminally truncated.

Using Hsp90α-pc3.1-Nhe1-For-Myc: GCTAGCTAGCGCCACCATGGA ACAAAAACTCATCTCAGAAGAGGATCTGCCTGAGGAAACCCAGACCCAAGAC (SEQ ID No. 10) and Hsp90α-pc3.1-Xho1-Re-nostop: CCCGCTCGA GTGTCTACTTCTTCCATGCGTGATG (SEQ ID No.12) as primers (synthesized by Invitrogen) and Pfu DNA polymerase (NEB), amplify Hsp90α from the template of pQE80L-Hsp90α (obtained in example 3). The products were digested and inserted into pcDNA3.1/Myc-His(-) (Invitrogen) to obtain Hsp90α with Myc tag at the N terminus, which was named as Myc-H. Similarly, His-Myc-H with N-terminal tandem His-Myc tags was amplified by Hsp90α-pc3.1-Nhe1-For-His-Myc: GCTAGCTAGCGCCACCATGCATCATCATCATCATCATGAACAAAAACTCATCTCAGA AGAGGATCTGCCTGAGGAAACCCAGACCCAAGAC (SEQ ID No.11) and SEQ ID No.12 primers. These two plasmids were transiently transfected into MCF-7 to observe the secretion of exogenous Hsp90α. Antibodies against Hsp90α, Myc and His were used to detect the secreted Hsp90α. The result shows that the secreted Hsp90α was C-terminally truncated (Fig.9A).

Using Myc-His-H as a template, the mutations of the last four amino acids (EEVD) of Hsp90α C-terminus was constructed. EE-> AA represents the mutation of two EE to two Ala (using SEQ ID No.11 and Hsp90α-EE- AA: GGCCGCTCGAGTGTCTACTGCTGCCATGCGTGATGTG (SEQ ID No.13) as primers), VD-> AA means that VD were mutated to two Ala (using SEQ ID No.11 and Hsp90α-VD-AA: GGCCGCTCGA GTTGCTGCTTCTTCCATGCGTGATGTG (SEQ ID No.14) as primers). All Ala represents the mutation of EEVD to four Ala (using SEQ ID No.11 and Hsp90α-EEVD-AAAA: GGCCGCTCGAGTTGCTGCTGCTGCCATGCG TGATGTG (SEQ ID No .15) as primers), CΔ4 represents the deletion of last EEVD four amino acids (using SEQ ID No.11 and Hsp90α-CΔ4-Xho: CCGCTCGAGTCATGCGTGATGTGTCGTCATCTC (SEQ ID No.16) as primers). Human breast cancer cell line MCF-7 was transiently transfected with these mutants to observe the secretion of exogenous Hsp90α (over-expressed Hsp90α, which is different from the endogenous one). The antibody against Hsp90α was used to detect the changes of secreted Hsp90α in the extracellular medium. The results show that the last four C-terminal amino acid residues regulate the secretion of Hsp90α. Any site-directed mutation or deletion of these four amino acid residues can lead to the secretion of Hsp90α without C-terminal truncation, which suggests that four C-terminal amino acid residues EEVD are deleted in the secreted extracellular Hsp90α (Figure 9B).

### Example 8: Examination of the existing form of Hsp90α in human plasma

We collected whole blood samples of liver cancer patients, which were centrifuged twice within 24 hours after collection, then detected Hsp90α in plasma using the method of immunoprecipitation and immunoblotting. First, specific rabbit polyclonal antibody against Hsp90α (Source: Labvision) was used for immunoprecipitation, and then a rabbit polyclonal antibody which can specifically recognize the Hsp90α C-terminal four amino acid residues EEVD (lab stock, antigen used for immunization is a carrier protein coupled with three-repeated peptides of EEVD, synthesized from the SBS Genetech Co,. Ltd.) was used to detect Hsp90α in plasma. As shown in Figure 10, the EEVD antibody specifically recognize Hsp90α from whole cell lysate, but does not recognize Hsp90α in plasma, which indicates plasma Hsp90α lacks the four C-terminal amino acid residues EEVD and is different from intracellular Hsp90α. (Figure 10).

### Example 9: Detection of the phosphorylated form of Hsp90α in plasma

Whole blood samples of the liver cancer patients were centrifuged twice to extract the plasma within 24 hours after collection. Then Hsp90α in plasma was detected using the method of immunoprecipitation and immunoblotting. First, specific rabbit polyclonal antibody against Hsp90α (Source: Labvision) was used to immunprecipitate plasma Hsp90α, then an antibody (Rabbit anti-phospho-(Ser/Thr) PKA substrate pAb, Cell signaling) which can specifically recognize the Thr90 phosphorylated Hsp90 was used to detect the Thr90 phosphorylation status of plasma Hsp90. As shown in Figure 11, the Hsp90α in plasma is Thr90 phosphorylated.

### Example 10: Detect the concentration of Thr90 phosphorylated Hsp90 in plasma

Whole blood samples of both liver cancer patients and normal people were centrifuged twice to extract the plasma within 24 hours after collection. The relative level of Hsp90α in the plasma was detected using the method of sandwich ELISA. Protocol: firstly, using self-made rabbit polycolonal antibody S2 to coat the plate overnight at 4□, then added 10-fold diluted plasma samples at 100 µl per well. After incubation at 37 □ for 2 hours, antibody which can specifically recognize the Thr90 phosphorylated Hsp90α (cell signal) was added. The plate is incubated at 37 □ for 2 hours; and then horseradish peroxidase conjugated goat anti-rabbit secondary antibodies were added and incubated for 1 hour. Finally o-phenylenediamine was added to detect the absorption at OD490nm. The results show that the levels of Hsp90α in liver cancer patients are higher than those of normal people, P=0.003, Student t-test, which indicates that the level of Thr90 phosphorylated Hsp90 is increased in liver cancer patients (Figure 12).

### Example 11: Detection of the consistency of Thr90 phosphorylated Hsp90 level and total Hsp90α level in plasma.

The levels of the Thr90 phosphorylated Hsp90 and the total amount of Hsp90α in liver cancer patients plasma (8 cases) were detected. The method to detect the total amount of plasma Hsp90α was the same as that used in example 5; the method to detect the level of Thr90 phosphorylated Hsp90 was the same as that used in example 10. The results show that the level of Thr90 phosphorylated plasma Hsp90 is consistent with the total amount of plasma Hsp90α, which further indicates that the phosphorylation of plasma Hsp90α is on Thr90, and the increment of the total amount of Hsp90α can represent the increment of Thr90 phosphorylated Hsp90α (Figure 13).

### Example 12: The phosphorylation of Thr90 is necessary for the secretion of Hsp90α

Using pcDNA3.1-Myc-His-Hsp90α plasmid as the template (Also known as wild-type Hsp90α (WT Hsp90α)), mutant Hsp90α (T90A, threonine mutated to alanine) was constructed by quickchange PCR using the following primers: Hsp90α-T89A-Sense:GATCGAACTCTTGCAATTGTGGATACTGGAATTGGAATG(SEQI D No.17) and Hsp90α-T89-AntiSense: CATTCCAATTCCAGTATCCACAATTGCAAGAGT TCGATC (SEQ ID No.18). T90A Hsp90α mutant cannot be phosphorylated at Thr90. Human breast cancer cell line MCF-7 (purchased from ATCC, No. HTB-22) was transfected with wild-type Hsp90α (WT) or mutant Hsp90α (T90A). The medium was then collected and the secretion of exogenous Hsp90α was detected by anti-Hsp90α, antibody.

The results show that overexpressed exogenous wild-type Hsp90α can be detected in the extracellular medium, while the T90A mutant can not be detected, which indicates that phosphorylation at residue Thr90 is a prerequisite for Hsp90α secretion (Figure 14).

### Example 13: PP5 is responsible for the dephosphorylation of pT90-Hsp90α

The preparation of the Thr90 phosphorylated Hsp90α (pT90-Hsp90α): The recombinant human Hsp90α protein and recombinant protein kinase A (Promega Corporation USA) were incubated in a reaction buffer (NEB UK company) at 30 °C for 1 h, then pT90-Hsp90α protein was purified. After removing free phosphate by dialysis, the purified pT90-Hsp90α protein mixed with recombinant human PP5 protein were incubated at 30 °C, the free phosphate released from the pT90-Hsp90α was detected using the non-radioactive serine/threonine phosphatase assay kit (Promega Corporation USA). Peptide substrate is a composition of the kit and was used as the positive control. As shown in Figure 15A, when PP5 was incubated with the peptide substrate, the release of free phosphate was significantly increased, P value <0.005, Student t test, indicating that PP5 can directly dephosphorylate the peptide substrate (positive control). When PP5 was incubated with pT90-Hsp90α protein, the release of free phosphate was also significantly increased, P value <0.005, Student t test, indicating that PP5 can directly dephosphorylate pT90-Hsp90α.

The nucleotide sequence of PP5 (SEQ ID No.6) was amplified from human liver cDNA library and constructed into the pcDNA3.1/Myc-His (-) (vector source: Invitrogen). The PP5 vector was transfected into and overexpressed in human breast cancer cell line MCF-7. On the other side, using the RNA interference technology, the expression of PP5 can also be knocked down using the siRNA with the sequence of 5'-ACTCGAACACCTCGCTAAAGAGCTC-3 '(SEQ ID No.7) (synthesized by Invitrogen). Then the status of Hsp90α Thr90 phosphorylation was examined with the overexpression or knock down of PP5. As shown in Figure 15B, with overexpression of human PP5, the Thr90 phosphorylated Hsp90α (pT90-Hsp90α) was significantly reduced (0.55 of the control). When the expression of human PP5 was suppressed, the Thr90 phosphorylated Hsp90α (pT90-Hsp90α) was significantly increased (1.58 of the control).

### Example 14: Regulation of the secretion of Hsp90α by promoting or inhibiting the expression of PP5

PP5 can dephosphorylate Thr90 phosphorylated Hsp90α. Primers as follows were used to amplify PP5 from human liver cDNA library: PP5-NheI-For: CTAGCTAGCATGTACCCATACGACGTCCCAGACTACGCT (SEQ ID No. 19) and PP5-XhoI-Re: CCGCTCGAGTTAATGATGATGATGATG ATGCACGTGTACC (SEQ ID No.20). The full length human PP5 was cloned into pcDNA3.1/Myc-His (-) (vector source: Invitrogen). Human breast cancer cell line MCF-7 was transfected with PP5 vector, then the secretion of Hsp90α from the cells was examined. The results showed that after overexpression of human PP5, the secretion of Hsp90α was significantly decreased (Figure 16A).

On the other side, when the expression of human PP5 in MCF-7 cells was knocked down by RNA interference (against human PP5, Invitrogen), the secretion of Hsp90α was significantly increased (Figure 16B).

### Example 15: The level of PP5 and the tumor malignancy

The relationship between the expression level of PP5 and the secretion of Hsp90α was examined in human breast cancer cell lines MCF-7, SKBR3, MDA-MB-453, 435s and 231 (ATCC, number, respectively HTB-22, -30, -131, -129, and HTB-26) using the method of western blotting. MCF-7, SKBR3 breast cancer cell lines are less malignant cell lines. In nude mice, these two cell lines can form primary tumors, but do not metastasize. MDA-MB-453, 435s and 231 are more malignant. They can not only form primary tumors, but also metastasize to distant organs in nude mice. Thus MDA-MB-435s and 231 are often used to establish tumor metastasis models.

Figure 17 shows that the cells, which express high level of PP5, secrete low level of Hsp90α; whereas cells with low expression of PP5 can secrete more Hsp90α. Meanwhile, the results also show that the level of secreted Hsp90α is positively correlated, whereas the expression level of PP5 is negatively correlated with the tumor malignancy (Figure 17), indicating that the level of secreted Hsp90α and its regulatory factors such as PP5 can be used to determine the tumor malignancy.

### Example 16: The expression level of PP5 is linked to tumor motility

The wound healing model was employed to examine the relationship between the expression level of PP5 and tumor cell migration.

Human breast cancer cell line MCF-7 was transfected with human PP5 vector or PP5 siRNA. Then the cells were inoculated into 12-well plate. When the cells grew to confluence, pipette tips were used to scrape cells to form a "wound". The scraped cells were washed away, and the rest cells were cultured in fresh DMEM medium (GIBCO) at 37 □ in an incubator with 5% of CO₂. The images of the "wound" at the time of 0 h, 12 h, and 24 h were captured (Figure 18A). The effect of PP5 expression on cell migration was examined by analyzing the "wound" healing rate. The results show that overexpression of PP5 inhibits MCF-7 cell migration, while PP5 siRNA can promote MCF-7 cell migration (Figure 18B).

### Example 17: The effect of plasma Hsp90α specific antibodies on tumor cell migration

The wound healing model was used to detect the effect of plasma Hsp90α specific antibody on tumor cell migration.

MCF-7 or MDA-MB-231 cells (ATCC, No. HTB-22 and HTB-26, respectively) were inoculated into 12-well plate. When the cells grow to confluence, pipette tips were used to scrape cells to form a "wound". The scraped cells were washed away, and the rest cells were cultured in fresh DMEM medium (GIBCO). Meanwhile, E9 (20 µg/ml), the specific mouse monoclonal antibody against Hsp90α, or control IgG (20 µg/ml ) was added. Then the plate was incubated at 37 □, with 5% CO₂, Images of the "wound" after 0 h, 6 h, 12 h, 24 h, 48 h, and 72 h of incubation were captured (Figure 18A). The effect of plasma Hsp90 specific antibodies on tumor cells migration was examined by monitoring the "wound" healing rate. As shown in Figure 19, the specific antibody against plasma Hsp90α can significantly inhibit the migration of both MDA-MB-231 (Figure 19A) and MCF-7 (Figure 19B) (Inhibitory rate > 40%).

### Example 18: Detect the effect of plasma Hsp90α specific antibody on tumor metastasis

Nude mice with an average body weight of 20 g were purchased from Beijing Vital River Laboratory Animal Technology Co., Ltd. B16/F10 mouse melanoma cells (ATCC, number: CRL-6475) (2×10⁵) were injected into the mice *via* tail vein. Next day the mice were randomly divided into two groups (n = 8): the control group (IgG was administered) and the Hsp90α Ab (mouse monoclonal antibody E9) treated group. The antibodies were administered once every other day with a dosage of 40 µg/mouse/time. Metastasis was examined 15 days after inoculation. As shown in Figure 20, the specific antibody against plasma Hsp90α can completely inhibit the lymph node metastasis of B16/F10 cells (A), and the lung metastasis can be inhibited by 56% (B).
## Claims

1. A method for determining the existence of malignant tumors or tumor metastasis which comprises measuring the Thr90 phosphorylated Hsp90α level of plasma samples from suspected tumor patients or suspected tumor metastasis patients.

2. The method according to claim 1, wherein the method uses an antibody specific for the Thr90 phosphorylated Hsp90α.

3. A method for screening high-risk populations for cancer, for predicting the prognosis of cancer patients, or for determining whether the treatment of surgery, radiotherapy or chemotherapy on cancer patients is effective, by detecting the level of plasma Thr90 phosphorylated Hsp90α in a sample.

4. The method of any one of claims 1-3, wherein said cancer is selected from the group consisting of lung cancer, liver cancer, gastric cancer, gastrointestinal cancer, esophagus cancer, osteosarcoma, pancreatic cancer, lymphoma, colorectal cancer, breast cancer, prostate cancer, oral cancer, nasopharyngeal cancer, cervical cancer, ovarian cancer, leukemia, malignant melanoma, sarcoma, renal cell carcinoma and cholangiocarcinoma.

5. An agent for use in a method of inhibiting cancer invasiveness and metastasis, comprising the step of inhibiting the phosphorylation of intracellular Hsp90α at Thr90 in cancer cells, wherein the agent is a nucleic acid molecule encoding protein phosphatase 5 in tumor cells.

6. The agent for use according to claim 5, wherein said use comprises overexpression of the nucleic acid molecule which is achieved by gene introduction.

7. The agent for use according to claim 5, wherein the protein phosphatase 5 comprises the amino acid sequence of SEQ ID No.5.

8. The agent for use according to any one of claims 5-7, wherein said cancer is selected from the group consisting of lung cancer, liver cancer, gastric cancer, gastrointestinal cancer, esophagus cancer, osteosarcoma, pancreatic cancer, lymphoma, colorectal cancer, breast cancer, prostate cancer, oral cancer, nasopharyngeal cancer, cervical cancer, ovarian cancer, leukemia, malignant melanoma, sarcoma, renal cell carcinoma and cholangiocarcinoma.

9. An isolated polypeptide comprising or consisting of the amino acid sequence of SEQ ID No.1, wherein the Thr90 in the amino acid sequence of SEQ ID NO:1 is phosphorylated.

## Patentansprüche

1. Verfahren zum Feststellen der Existenz eines/einer bösartigen Tumors oder Tumor-Metastase, welches Messen des Thr90-phosphorylierten Hsp90α-Levels in Plasma-Proben von vermuteten Tumor-Patienten oder vermuteten Tumor-Metastase-Patienten umfasst.

2. Verfahren gemäß Anspruch 1, wobei im Verfahren ein Antikörper verwendet wird, welcher für das Thr90-phosphorylierte Hsp90α spezifisch ist.

3. Verfahren zum Screenen von Krebs-Hochrisiko-Populationen, um die Prognose von Krebs-Patienten zu prognostizieren, oder um zu festzustellen ob die Behandlung mittels Operation, Radiotherapie oder Chemotherapie an Krebspatienten effektiv ist, durch das Detektieren des Plasma-Levels von Thr90-phosphoryliertem Hsp90α in einer Probe.

4. Verfahren gemäß irgendeinem der Ansprüche 1-3, wobei der Krebs ausgewählt wird aus der Gruppe bestehend aus Lungenkrebs, Leberkrebs, Magenkrebs, Magen-Darm-Krebs, Speiseröhrenkrebs, Osteosarkom, Bauchspeicheldrüsenkrebs, Lymphom, Darmkrebs, Brustkrebs, Prostatakrebs, Mundkrebs, Nasopharynxkarzinoma, Gebärmutterhalskrebs, Eierstockkrebs, Leukämie, bösartiges Melanom, Sarkom, Nierenzellkarzinom und Gallengangskarzinom.

5. Agens zur Verwendung in einem Verfahren zur Inhibition von Krebs-Invasivität und Metastase, umfassend den Schritt des Inhibierens der Phosphorylierung an Thr90 von intrazellulärem Hsp90α in Krebszellen, wobei das Agens ein Nukleinsäuremolekül ist, welches Protein-Phosphatase 5 in Tumorzellen codiert.

6. Agens zur Verwendung gemäß Anspruch 5, wobei die Verwendung Überexprimierung des Nukleinsäuremoleküls, welche durch Gen-Einführung erzielt wird, umfasst.

7. Agens zur Verwendung gemäß Anspruch 5, wobei die Protein-Phosphatase 5 die Aminosäuresequenz von SEQ ID No. 5 umfasst.

8. Agens zur Verwendung gemäß irgendeinem der Ansprüche 5-7, wobei der Krebs ausgewählt wird aus der Gruppe bestehend aus Lungenkrebs, Leberkrebs, Magenkrebs, Magen-Darm-Krebs, Speiseröhrenkrebs, Osteosarkom, Bauchspeicheldrüsenkrebs, Lymphom, Darmkrebs, Brustkrebs, Prostatakrebs, Mundkrebs, Nasopharynxkarzinoma, Gebärmutterhalskrebs, Eierstockkrebs, Leukämie, bösartiges Melanom, Sarkom, Nierenzellkarzinom und Gallengangskarzinom.

9. Isoliertes Polypeptid, umfassend oder bestehend aus der Aminosäuresequenz von SEQ ID No. 1, wobei das Thr90 in der Aminosäuresequenz von SEQ ID No. 1 phosphoryliert ist.

## Revendications

1. Procédé pour déterminer l'existence de tumeurs malignes ou de métastases tumorales, qui comprend la mesure du taux de Hsp90α phosphorylée sur Thr90 d'échantillons de plasma provenant de patients soupçonnés d'être atteints d'une tumeur ou de patients soupçonnés d'être atteints de métastases tumorales.

2. Procédé selon la revendication 1, le procédé utilisant un anticorps spécifique de la Hsp90α phosphorylée sur Thr90.

3. Procédé pour dépister un cancer chez des populations à risque élevé, pour prévoir le pronostic des patients cancéreux ou pour déterminer si le traitement de chirurgie, de radiothérapie ou de chimiothérapie sur des patients cancéreux est efficace, en détectant le taux de Hsp90α phosphorylée sur Thr90 dans un échantillon.

4. Procédé selon l'une quelconque des revendications 1-3, ledit cancer étant choisi dans le groupe constitué du cancer du poumon, du cancer du foie, du cancer gastrique, du cancer gastro-intestinal, du cancer de l'oesophage, de l'ostéosarcome, du cancer du pancréas, du lymphome, du cancer colorectal, du cancer du sein, du cancer de la prostate, du cancer buccal, du cancer du nasopharynx, du cancer du col de l'utérus, du cancer des ovaires, de la leucémie, du mélanome malin, du sarcome, du carcinome à cellules rénales et du cholangiocarcinome.

5. Agent pour une utilisation dans un procédé destiné à inhiber le caractère invasif d'un cancer et ses métastases, comprenant l'étape consistant à inhiber la phosphorylation de la Hsp90α intracellulaire sur Thr90 dans des cellules cancéreuses, l'agent étant une molécule d'acide nucléique codant pour la protéine phosphatase 5 dans des cellules cancéreuses.

6. Agent pour une utilisation selon la revendication 5, ladite utilisation comprenant la surexpression de la molécule d'acide nucléique qui est obtenue par l'introduction d'un gène.

7. Agent pour une utilisation selon la revendication 5, la protéine phosphatase 5 comprenant la séquence d'acides aminés SEQ ID n° 5.

8. Agent pour une utilisation selon l'une quelconque des revendications 5-7, ledit cancer étant choisi dans le groupe constitué du cancer du poumon, du cancer du foie, du cancer gastrique, du cancer gastro-intestinal, du cancer de l'oesophage, de l'ostéosarcome, du cancer du pancréas, du lymphome, du cancer colorectal, du cancer du sein, du cancer de la prostate, du cancer buccal, du cancer du nasopharynx, du cancer du col de l'utérus, du cancer des ovaires, de la leucémie, du mélanome malin, du sarcome, du carcinome à cellules rénales et du cholangiocarcinome.

9. Polypeptide isolé comprenant la séquence d'acides aminés SEQ ID n° 1 ou constitué de ladite séquence, la Thr90 de la séquence d'acides aminés SEQ ID n° 1 étant phosphorylée.


<110> Tsinghua University
   Protgen Ltd
<120> A New Tumor Biomarker
<130> P2010653C
<140> EP 10796746.5
   <141> 2010-08-11
<150> CN200910158747.9
   <151> 2009-07-07
<160> 20
<170> PatentIn version 3.3
<210> 1
   <211> 728
   <212> PRT
   <213> Homo sapiens
<400> 1
   Met Pro Glu Glu Thr Gln Thr Gln Asp Gln Pro Met Glu Glu Glu Glu
   1               5                   10                  15
   Val Glu Thr Phe Ala Phe Gln Ala Glu Ile Ala Gln Leu Met Ser Leu
               20                  25                  30
   Ile Ile Asn Thr Phe Tyr Ser Asn Lys Glu Ile Phe Leu Arg Glu Leu
           35                  40                  45
   Ile Ser Asn Ser Ser Asp Ala Leu Asp Lys Ile Arg Tyr Glu Thr Leu
       50                  55                  60
   Thr Asp Pro Ser Lys Leu Asp Ser Gly Lys Glu Leu His Ile Asn Leu
   65                  70                  75                  80
   Ile Pro Asn Lys Gln Asp Arg Thr Leu Thr Ile Val Asp Thr Gly Ile
                   85                  90                  95
   Gly Met Thr Lys Ala Asp Leu Ile Asn Asn Leu Gly Thr Ile Ala Lys
               100                 105                 110
   Ser Gly Thr Lys Ala Phe Met Glu Ala Leu Gln Ala Gly Ala Asp Ile
           115                 120                 125
   Ser Met Ile Gly Gln Phe Gly Val Gly Phe Tyr Ser Ala Tyr Leu Val
       130                 135                 140
   Ala Glu Lys Val Thr Val Ile Thr Lys His Asn Asp Asp Glu Gln Tyr
   145                 150                 155                 160
   Ala Trp Glu Ser Ser Ala Gly Gly Ser Phe Thr Val Arg Thr Asp Thr
                   165                 170                 175
   Gly Glu Pro Met Gly Arg Gly Thr Lys Val Ile Leu His Leu Lys Glu
                   180                 185                 190
   Asp Gln Thr Glu Tyr Leu Glu Glu Arg Arg Ile Lys Glu Ile Val Lys
           195                 200                 205
   Lys His Ser Gln Phe Ile Gly Tyr Pro Ile Thr Leu Phe Val Glu Lys
       210                 215                 220
   Glu Arg Asp Lys Glu Val Ser Asp Asp Glu Ala Glu Glu Lys Glu Asp
   225                 230                 235                 240
   Lys Glu Glu Glu Lys Glu Lys Glu Glu Lys Glu Ser Glu Asp Lys Pro
                   245                 250                 255
   Glu Ile Glu Asp Val Gly Ser Asp Glu Glu Glu Glu Lys Lys Asp Gly
               260                 265                 270
   Asp Lys Lys Lys Lys Lys Lys Ile Lys Glu Lys Tyr Ile Asp Gln Glu
           275                 280                 285
   Glu Leu Asn Lys Thr Lys Pro Ile Trp Thr Arg Asn Pro Asp Asp Ile
       290                 295                 300
   Thr Asn Glu Glu Tyr Gly Glu Phe Tyr Lys Ser Leu Thr Asn Asp Trp
   305                 310                 315                 320
   Glu Asp His Leu Ala Val Lys His Phe Ser Val Glu Gly Gln Leu Glu
                   325                 330                 335
   Phe Arg Ala Leu Leu Phe Val Pro Arg Arg Ala Pro Phe Asp Leu Phe
               340                 345                 350
   Glu Asn Arg Lys Lys Lys Asn Asn Ile Lys Leu Tyr Val Arg Arg Val
           355                 360                 365
   Phe Ile Met Asp Asn Cys Glu Glu Leu Ile Pro Glu Tyr Leu Asn Phe
       370                 375                 380
   Ile Arg Gly Val Val Asp Ser Glu Asp Leu Pro Leu Asn Ile Ser Arg
   385                 390                 395                 400
   Glu Met Leu Gln Gln Ser Lys Ile Leu Lys Val Ile Arg Lys Asn Leu
                   405                 410                 415
   Val Lys Lys Cys Leu Glu Leu Phe Thr Glu Leu Ala Glu Asp Lys Glu
               420                 425                 430
   Asn Tyr Lys Lys Phe Tyr Glu Gln Phe Ser Lys Asn Ile Lys Leu Gly
           435                 440                 445
   Ile His Glu Asp Ser Gln Asn Arg Lys Lys Leu Ser Glu Leu Leu Arg
       450                 455                 460
   Tyr Tyr Thr Ser Ala Ser Gly Asp Glu Met Val Ser Leu Lys Asp Tyr
   465                 470                 475                 480
   Cys Thr Arg Met Lys Glu Asn Gln Lys His Ile Tyr Tyr Ile Thr Gly
                   485                 490                 495
   Glu Thr Lys Asp Gln Val Ala Asn Ser Ala Phe Val Glu Arg Leu Arg
               500                 505                 510
   Lys His Gly Leu Glu Val Ile Tyr Met Ile Glu Pro Ile Asp Glu Tyr
           515                 520                 525
   Cys Val Gln Gln Leu Lys Glu Phe Glu Gly Lys Thr Leu Val Ser Val
       530                 535                 540
   Thr Lys Glu Gly Leu Glu Leu Pro Glu Asp Glu Glu Glu Lys Lys Lys
   545                 550                 555                 560
   Gln Glu Glu Lys Lys Thr Lys Phe Glu Asn Leu Cys Lys Ile Met Lys
                   565                 570                 575
   Asp Ile Leu Glu Lys Lys Val Glu Lys Val Val Val Ser Asn Arg Leu
               580                 585                 590
   Val Thr Ser Pro Cys Cys Ile Val Thr Ser Thr Tyr Gly Trp Thr Ala
           595                 600                 605
   Asn Met Glu Arg Ile Met Lys Ala Gln Ala Leu Arg Asp Asn Ser Thr
       610                 615                 620
   Met Gly Tyr Met Ala Ala Lys Lys His Leu Glu Ile Asn Pro Asp His
   625                 630                 635                 640
   Ser Ile Ile Glu Thr Leu Arg Gln Lys Ala Glu Ala Asp Lys Asn Asp
                   645                 650                 655
   Lys Ser Val Lys Asp Leu Val Ile Leu Leu Tyr Glu Thr Ala Leu Leu
               660                 665                 670
   Ser Ser Gly Phe Ser Leu Glu Asp Pro Gln Thr His Ala Asn Arg Ile
           675                 680                 685
   Tyr Arg Met Ile Lys Leu Gly Leu Gly Ile Asp Glu Asp Asp Pro Thr
       690                 695                 700
   Ala Asp Asp Thr Ser Ala Ala Val Thr Glu Glu Met Pro Pro Leu Glu
   705 710 715 720
   Gly Asp Asp Asp Thr Ser Arg Met
   725
<210> 2
   <211> 2184
   <212> DNA
   <213> Homo sapiens
<400> 2
   atgcctgagg aaacccagac ccaagaccaa ccgatggagg aggaggaggt tgagacgttc    60
   gcctttcagg cagaaattgc ccagttgatg tcattgatca tcaatacttt ctactcgaac   120
   aaagagatct ttctgagaga gctcatttca aattcatcag atgcattgga caaaatccgg   180
   tatgaaactt tgacagatcc cagtaaatta gactctggga aagagctgca tattaacctt   240
   ataccgaaca aacaagatcg aactctcact attgtggata ctggaattgg aatgaccaag   300
   gctgacttga tcaataacct tggtactatc gccaagtctg ggaccaaagc gttcatggaa   360
   gctttgcagg ctggtgcaga tatctctatg attggccagt tcggtgttgg tttttattct   420
   gcttatttgg ttgctgagaa agtaactgtg atcaccaaac ataacgatga tgagcagtac   480
   gcttgggagt cctcagcagg gggatcattc acagtgagga cagacacagg tgaacctatg   540
   ggtcgtggaa caaaagttat cctacacctg aaagaagacc aaactgagta cttggaggaa   600
   cgaagaataa aggagattgt gaagaaacat tctcagttta ttggatatcc cattactctt   660
   tttgtggaga aggaacgtga taaagaagta agcgatgatg aggctgaaga aaaggaagac   720
   aaagaagaag aaaaagaaaa agaagagaaa gagtcggaag acaaacctga aattgaagat   780
   gttggttctg atgaggaaga agaaaagaag gatggtgaca agaagaagaa gaagaagatt   840
   aaggaaaagt acatcgatca agaagagctc aacaaaacaa agcccatctg gaccagaaat   900
   cccgacgata ttactaatga ggagtacgga gaattctata agagcttgac caatgactgg   960
   gaagatcact tggcagtgaa gcatttttca gttgaaggac agttggaatt cagagccctt  1020
   ctatttgtcc cacgacgtgc tccttttgat ctgtttgaaa acagaaagaa aaagaacaat  1080
   atcaaattgt atgtacgcag agttttcatc atggataact gtgaggagct aatccctgaa  1140
   tatctgaact tcattagagg ggtggtagac tcggaggatc tccctctaaa catatcccgt  1200
   gagatgttgc aacaaagcaa aattttgaaa gttatcagga agaatttggt caaaaaatgc  1260
   ttagaactct ttactgaact ggcggaagat aaagagaact acaagaaatt ctatgagcag  1320
   ttctctaaaa acataaagct tggaatacac gaagactctc aaaatcggaa gaagctttca  1380
   gagctgttaa ggtactacac atctgcctct ggtgatgaga tggtttctct caaggactac  1440
   tgcaccagaa tgaaggagaa ccagaaacat atctattata tcacaggtga gaccaaggac  1500
   caggtagcta actcagcctt tgtggaacgt cttcggaaac atggcttaga agtgatctat   1560
   atgattgagc ccattgatga gtactgtgtc caacagctga aggaatttga ggggaagact   1620
   ttagtgtcag tcaccaaaga aggcctggaa cttccagagg atgaagaaga gaaaaagaag   1680
   caggaagaga aaaaaacaaa gtttgagaac ctctgcaaaa tcatgaaaga catattggag   1740
   aaaaaagttg aaaaggtggt tgtgtcaaac cgattggtga catctccatg ctgtattgtc   1800
   acaagcacat atggctggac agcaaacatg gagagaatca tgaaagctca agccctaaga   1860
   gacaactcaa caatgggtta catggcagca aagaaacacc tggagataaa ccctgaccat   1920
   tccattattg agaccttaag gcaaaaggca gaggctgata agaacgacaa gtctgtgaag   1980
   gatctggtca tcttgcttta tgaaactgcg ctcctgtctt ctggcttcag tctggaagat   2040
   ccccagacac atgctaacag gatctacagg atgatcaaac ttggtctggg tattgatgaa   2100
   gatgacccta ctgctgatga taccagtgct gctgtaactg aagaaatgcc accccttgaa   2160
   ggagatgacg acacatcacg catg                                          2184
<210> 3
   <211> 732
   <212> PRT
   <213> Homo sapiens
<400> 3
   Met Pro Glu Glu Thr Gln Thr Gln Asp Gln Pro Met Glu Glu Glu Glu
   1               5                   10                  15
   Val Glu Thr Phe Ala Phe Gln Ala Glu Ile Ala Gln Leu Met Ser Leu
               20                  25                  30
   Ile Ile Asn Thr Phe Tyr Ser Asn Lys Glu Ile Phe Leu Arg Glu Leu
           35                  40                  45
   Ile Ser Asn Ser Ser Asp Ala Leu Asp Lys Ile Arg Tyr Glu Thr Leu
       50                  55                  60
   Thr Asp Pro Ser Lys Leu Asp Ser Gly Lys Glu Leu His Ile Asn Leu
   65                  70                  75                  80
   Ile Pro Asn Lys Gln Asp Arg Thr Leu Thr Ile Val Asp Thr Gly Ile
                   85                  90                  95
   Gly Met Thr Lys Ala Asp Leu Ile Asn Asn Leu Gly Thr Ile Ala Lys
               100                 105                 110
   Ser Gly Thr Lys Ala Phe Met Glu Ala Leu Gln Ala Gly Ala Asp Ile
           115                 120                 125
   Ser Met Ile Gly Gln Phe Gly Val Gly Phe Tyr Ser Ala Tyr Leu Val
           130                 135                 140
   Ala Glu Lys Val Thr Val Ile Thr Lys His Asn Asp Asp Glu Gln Tyr
   145                 150                 155                 160
   Ala Trp Glu Ser Ser Ala Gly Gly Ser Phe Thr Val Arg Thr Asp Thr
                   165                 170                 175
   Gly Glu Pro Met Gly Arg Gly Thr Lys Val Ile Leu His Leu Lys Glu
               180                 185                 190
   Asp Gln Thr Glu Tyr Leu Glu Glu Arg Arg Ile Lys Glu Ile Val Lys
           195                 200                 205
   Lys His Ser Gln Phe Ile Gly Tyr Pro Ile Thr Leu Phe Val Glu Lys
       210                 215                 220
   Glu Arg Asp Lys Glu Val Ser Asp Asp Glu Ala Glu Glu Lys Glu Asp
   225                 230                 235                 240
   Lys Glu Glu Glu Lys Glu Lys Glu Glu Lys Glu Ser Glu Asp Lys Pro
                   245                 250                 255
   Glu Ile Glu Asp Val Gly Ser Asp Glu Glu Glu Glu Lys Lys Asp Gly
               260                 265                 270
   Asp Lys Lys Lys Lys Lys Lys Ile Lys Glu Lys Tyr Ile Asp Gln Glu
           275                 280                 285
   Glu Leu Asn Lys Thr Lys Pro Ile Trp Thr Arg Asn Pro Asp Asp Ile
       290                 295                 300
   Thr Asn Glu Glu Tyr Gly Glu Phe Tyr Lys Ser Leu Thr Asn Asp Trp
   305                 310                 315                 320
   Glu Asp His Leu Ala Val Lys His Phe Ser Val Glu Gly Gln Leu Glu
                   325                 330                 335
   Phe Arg Ala Leu Leu Phe Val Pro Arg Arg Ala Pro Phe Asp Leu Phe
               340                 345                 350
   Glu Asn Arg Lys Lys Lys Asn Asn Ile Lys Leu Tyr Val Arg Arg Val
           355                 360                 365
   Phe Ile Met Asp Asn Cys Glu Glu Leu Ile Pro Glu Tyr Leu Asn Phe
       370                 375                 380
   Ile Arg Gly Val Val Asp Ser Glu Asp Leu Pro Leu Asn Ile Ser Arg
   385                 390                 395                 400
   Glu Met Leu Gln Gln Ser Lys Ile Leu Lys Val Ile Arg Lys Asn Leu
                   405                 410                 415
   Val Lys Lys Cys Leu Glu Leu Phe Thr Glu Leu Ala Glu Asp Lys Glu
               420                 425                 430
   Asn Tyr Lys Lys Phe Tyr Glu Gln Phe Ser Lys Asn Ile Lys Leu Gly
           435                 440                 445
   Ile His Glu Asp Ser Gln Asn Arg Lys Lys Leu Ser Glu Leu Leu Arg
       450                 455                 460
   Tyr Tyr Thr Ser Ala Ser Gly Asp Glu Met Val Ser Leu Lys Asp Tyr
   465                 470                 475                 480
   Cys Thr Arg Met Lys Glu Asn Gln Lys His Ile Tyr Tyr Ile Thr Gly
                   485                 490                 495
   Glu Thr Lys Asp Gln Val Ala Asn Ser Ala Phe Val Glu Arg Leu Arg
               500                 505                 510
   Lys His Gly Leu Glu Val Ile Tyr Met Ile Glu Pro Ile Asp Glu Tyr
           515                 520                 525
   Cys Val Gln Gln Leu Lys Glu Phe Glu Gly Lys Thr Leu Val Ser Val
       530                 535                 540
   Thr Lys Glu Gly Leu Glu Leu Pro Glu Asp Glu Glu Glu Lys Lys Lys
   545                 550                 555                 560
   Gln Glu Glu Lys Lys Thr Lys Phe Glu Asn Leu Cys Lys Ile Met Lys
                   565                 570                 575
   Asp Ile Leu Glu Lys Lys Val Glu Lys Val Val Val Ser Asn Arg Leu
               580                 585                 590
   Val Thr Ser Pro Cys Cys Ile Val Thr Ser Thr Tyr Gly Trp Thr Ala
           595                 600                 605
   Asn Met Glu Arg Ile Met Lys Ala Gln Ala Leu Arg Asp Asn Ser Thr
       610                 615                 620
   Met Gly Tyr Met Ala Ala Lys Lys His Leu Glu Ile Asn Pro Asp His
   625                 630                 635                 640
   Ser Ile Ile Glu Thr Leu Arg Gln Lys Ala Glu Ala Asp Lys Asn Asp
                   645                 650                 655
   Lys Ser Val Lys Asp Leu Val Ile Leu Leu Tyr Glu Thr Ala Leu Leu
               660                 665                 670
   Ser Ser Gly Phe Ser Leu Glu Asp Pro Gln Thr His Ala Asn Arg Ile
           675                 680                 685
   Tyr Arg Met Ile Lys Leu Gly Leu Gly Ile Asp Glu Asp Asp Pro Thr
       690                 695                 700
   Ala Asp Asp Thr Ser Ala Ala Val Thr Glu Glu Met Pro Pro Leu Glu
   705                 710                 715                 720
   Gly Asp Asp Asp Thr Ser Arg Met Glu Glu Val Asp
                   725                 730
<210> 4
   <211> 2199
   <212> DNA
   <213> Homo sapiens
<400> 4
   atgcctgagg aaacccagac ccaagaccaa ccgatggagg aggaggaggt tgagacgttc      60
   gcctttcagg cagaaattgc ccagttgatg tcattgatca tcaatacttt ctactcgaac     120
   aaagagatct ttctgagaga gctcatttca aattcatcag atgcattgga caaaatccgg     180
   tatgaaactt tgacagatcc cagtaaatta gactctggga aagagctgca tattaacctt     240
   ataccgaaca aacaagatcg aactctcact attgtggata ctggaattgg aatgaccaag     300
   gctgacttga tcaataacct tggtactatc gccaagtctg ggaccaaagc gttcatggaa     360
   gctttgcagg ctggtgcaga tatctctatg attggccagt tcggtgttgg tttttattct     420
   gcttatttgg ttgctgagaa agtaactgtg atcaccaaac ataacgatga tgagcagtac     480
   gcttgggagt cctcagcagg gggatcattc acagtgagga cagacacagg tgaacctatg     540
   ggtcgtggaa caaaagttat cctacacctg aaagaagacc aaactgagta cttggaggaa     600
   cgaagaataa aggagattgt gaagaaacat tctcagttta ttggatatcc cattactctt     660
   tttgtggaga aggaacgtga taaagaagta agcgatgatg aggctgaaga aaaggaagac     720
   aaagaagaag aaaaagaaaa agaagagaaa gagtcggaag acaaacctga aattgaagat     780
   gttggttctg atgaggaaga agaaaagaag gatggtgaca agaagaagaa gaagaagatt     840
   aaggaaaagt acatcgatca agaagagctc aacaaaacaa agcccatctg gaccagaaat     900
   cccgacgata ttactaatga ggagtacgga gaattctata agagcttgac caatgactgg     960
   gaagatcact tggcagtgaa gcatttttca gttgaaggac agttggaatt cagagccctt    1020
   ctatttgtcc cacgacgtgc tccttttgat ctgtttgaaa acagaaagaa aaagaacaat    1080
   atcaaattgt atgtacgcag agttttcatc atggataact gtgaggagct aatccctgaa    1140
   tatctgaact tcattagagg ggtggtagac tcggaggatc tccctctaaa catatcccgt 1200
   gagatgttgc aacaaagcaa aattttgaaa gttatcagga agaatttggt caaaaaatgc 1260
   ttagaactct ttactgaact ggcggaagat aaagagaact acaagaaatt ctatgagcag 1320
   ttctctaaaa acataaagct tggaatacac gaagactctc aaaatcggaa gaagctttca 1380
   gagctgttaa ggtactacac atctgcctct ggtgatgaga tggtttctct caaggactac 1440
   tgcaccagaa tgaaggagaa ccagaaacat atctattata tcacaggtga gaccaaggac 1500
   caggtagcta actcagcctt tgtggaacgt cttcggaaac atggcttaga agtgatctat 1560
   atgattgagc ccattgatga gtactgtgtc caacagctga aggaatttga ggggaagact 1620
   ttagtgtcag tcaccaaaga aggcctggaa cttccagagg atgaagaaga gaaaaagaag 1680
   caggaagaga aaaaaacaaa gtttgagaac ctctgcaaaa tcatgaaaga catattggag 1740
   aaaaaagttg aaaaggtggt tgtgtcaaac cgattggtga catctccatg ctgtattgtc 1800
   acaagcacat atggctggac agcaaacatg gagagaatca tgaaagctca agccctaaga 1860
   gacaactcaa caatgggtta catggcagca aagaaacacc tggagataaa ccctgaccat 1920
   tccattattg agaccttaag gcaaaaggca gaggctgata agaacgacaa gtctgtgaag 1980
   gatctggtca tcttgcttta tgaaactgcg ctcctgtctt ctggcttcag tctggaagat 2040
   ccccagacac atgctaacag gatctacagg atgatcaaac ttggtctggg tattgatgaa 2100
   gatgacccta ctgctgatga taccagtgct gctgtaactg aagaaatgcc accccttgaa 2160
   ggagatgacg acacatcacg catggaagaa gtagactaa 2199
<210> 5
   <211> 499
   <212> PRT
   <213> Homo sapiens
<400> 5
   Met Ala Met Ala Glu Gly Glu Arg Thr Glu Cys Ala Glu Pro Pro Arg
   1               5                   10                  15
   Asp Glu Pro Pro Ala Asp Gly Ala Leu Lys Arg Ala Glu Glu Leu Lys
               20                  25                  30
   Thr Gln Ala Asn Asp Tyr Phe Lys Ala Lys Asp Tyr Glu Asn Ala Ile
           35                  40                  45
   Lys Phe Tyr Ser Gln Ala Ile Glu Leu Asn Pro Ser Asn Ala Ile Tyr
       50                  55                  60
   Tyr Gly Asn Arg Ser Leu Ala Tyr Leu Arg Thr Glu Cys Tyr Gly Tyr
   65                  70                  75                  80
   Ala Leu Gly Asp Ala Thr Arg Ala Ile Glu Leu Asp Lys Lys Tyr Ile
                   85                  90                  95
   Lys Gly Tyr Tyr Arg Arg Ala Ala Ser Asn Met Ala Leu Gly Lys Phe
               100                 105                 110
   Arg Ala Ala Leu Arg Asp Tyr Glu Thr Val Val Lys Val Lys Pro His
           115                 120                 125
   Asp Lys Asp Ala Lys Met Lys Tyr Gln Glu Cys Asn Lys Ile Val Lys
       130                 135                 140
   Gln Lys Ala Phe Glu Arg Ala Ile Ala Gly Asp Glu His Lys Arg Ser
   145                 150                 155                 160
   Val Val Asp Ser Leu Asp Ile Glu Ser Met Thr Ile Glu Asp Glu Tyr
                   165                 170                 175
   Ser Gly Pro Lys Leu Glu Asp Gly Lys Val Thr Ile Ser Phe Met Lys
               180                 185                 190
   Glu Leu Met Gln Trp Tyr Lys Asp Gln Lys Lys Leu His Arg Lys Cys
           195                 200                 205
   Ala Tyr Gln Ile Leu Val Gln Val Lys Glu Val Leu Ser Lys Leu Ser
       210                 215                 220
   Thr Leu Val Glu Thr Thr Leu Lys Glu Thr Glu Lys Ile Thr Val Cys
   225                 230                 235                 240
   Gly Asp Thr His Gly Gln Phe Tyr Asp Leu Leu Asn Ile Phe Glu Leu
                   245                 250                 255
   Asn Gly Leu Pro Ser Glu Thr Asn Pro Tyr Ile Phe Asn Gly Asp Phe
               260                 265                 270
   Val Asp Arg Gly Ser Phe Ser Val Glu Val Ile Leu Thr Leu Phe Gly
           275                 280                 285
   Phe Lys Leu Leu Tyr Pro Asp His Phe His Leu Leu Arg Gly Asn His
       290                 295                 300
   Glu Thr Asp Asn Met Asn Gln Ile Tyr Gly Phe Glu Gly Glu Val Lys
   305                 310                 315                 320
   Ala Lys Tyr Thr Ala Gln Met Tyr Glu Leu Phe Ser Glu Val Phe Glu
                   325                 330                 335
   Trp Leu Pro Leu Ala Gln Cys Ile Asn Gly Lys Val Leu Ile Met His
               340                 345                 350
   Gly Gly Leu Phe Ser Glu Asp Gly Val Thr Leu Asp Asp Ile Arg Lys
           355                 360                 365
   Ile Glu Arg Asn Arg Gln Pro Pro Asp Ser Gly Pro Met Cys Asp Leu
       370                 375                 380
   Leu Trp Ser Asp Pro Gln Pro Gln Asn Gly Arg Ser Ile Ser Lys Arg
   385                 390                 395                 400
   Gly Val Ser Cys Gln Phe Gly Pro Asp Val Thr Lys Ala Phe Leu Glu
                   405                 410                 415
   Glu Asn Asn Leu Asp Tyr Ile Ile Arg Ser His Glu Val Lys Ala Glu
               420                 425                 430
   Gly Tyr Glu Val Ala His Gly Gly Arg Cys Val Thr Val Phe Ser Ala
           435                 440                 445
   Pro Asn Tyr Cys Asp Gln Met Gly Asn Lys Ala Ser Tyr Ile His Leu
       450                 455                 460
   Gln Gly Ser Asp Leu Arg Pro Gln Phe His Gln Phe Thr Ala Val Pro
   465                 470                 475                 480
   His Pro Asn Val Lys Pro Met Ala Tyr Ala Asn Thr Leu Leu Gln Leu
                   485                 490                 495
   Gly Met Met
<210> 6
   <211> 1500
   <212> DNA
   <213> Homo sapiens
<400> 6
   atggcgatgg cggagggcga gaggactgag tgtgctgagc ccccccggga cgaacccccg 60
   gctgatggag ctctgaagcg ggcagaggag ctcaagactc aggccaatga ctacttcaaa 120
   gccaaggact acgagaacgc catcaagttc tacagccagg ccatcgagct gaaccccagc 180
   aatgccatct actatggcaa ccgcagcctg gcctacctgc gcactgagtg ctatggctac 240
   gcgctgggag acgccacgcg ggccattgag ctggacaaga agtacatcaa gggttattac 300
   cgccgggctg ccagcaacat ggcactgggc aagttccggg ccgcgctgcg agactacgag 360
   acggtggtca aggtgaagcc ccatgacaag gatgccaaaa tgaaatacca ggagtgcaac 420
   aagatcgtga agcagaaggc ctttgagcgg gccatcgcgg gcgacgagca caagcgctcc 480
   gtggtggact cgctggacat cgagagcatg accattgagg atgagtacag cggacccaag 540
   cttgaagacg gcaaagtgac aatcagtttc atgaaggagc tcatgcagtg gtacaaggac 600
   cagaagaaac tgcaccggaa atgtgcctac cagattctgg tacaggtcaa agaggtcctc 660
   tccaagctga gcacgctcgt ggaaaccaca ctcaaagaga cagagaagat tacagtatgt 720
   ggggacaccc atggccagtt ctatgacctc ctcaacatat tcgagctcaa cggtttaccc 780
   tcggagacca acccctatat atttaatggt gactttgtgg accgaggctc cttctctgta 840
   gaagtgatcc tcaccctttt cggcttcaag ctcctgtacc cagatcactt tcacctcctt 900
   cgaggcaacc acgagacaga caacatgaac cagatctacg gtttcgaggg tgaggtgaag 960
   gccaagtaca cagcccagat gtacgagctc tttagcgagg tgttcgagtg gctcccgttg 1020
   gcccagtgca tcaacggcaa agtgctgatc atgcacggag gcctgttcag tgaagacggt 1080
   gtcaccctgg atgacatccg gaaaattgag cggaatcgac aacccccaga ttcagggccc 1140
   atgtgtgacc tgctctggtc agatccacag ccacagaacg ggcgctcgat cagcaagcgg 1200
   ggcgtgagct gtcagtttgg gcctgacgtc accaaggcct tcttggaaga gaacaacctg 1260
   gactatatca tccgcagcca cgaagtcaag gccgagggct acgaggtggc tcacggaggc 1320
   cgctgtgtca ccgtcttctc tgcccccaac tactgcgacc agatggggaa caaagcctcc 1380
   tacatccacc tccagggctc tgacctacgg cctcagttcc accagttcac agcagtgcct 1440
   catcccaacg tcaagcccat ggcctatgcc aacacgctgc tgcagctagg aatgatgtga 1500
<210> 7
   <211> 25
   <212> DNA
   <213> Artificial
<220>
   <223> Target sequence of PP5 siRNA
<400> 7
   actcgaacac ctcgctaaag agctc 25
<210> 8
   <211> 30
   <212> DNA
   <213> Artificial
<220>
   <223> Hsp90¦Á-Sal1-Re
<400> 8
   acgcgtcgac ttagtctact tcttccatgc 30
<210> 9
   <211> 31
   <212> DNA
   <213> Artificial
<220>
   <223> Hsp90¦Á-Sph1-For
<400> 9
   acatgcatgc atgcctgagg aaacccagac c 31
<210> 10
   <211> 73
   <212> DNA
   <213> Artificial
<220>
   <223> Hsp90¦Á-pc3.1-Nhe1-For-Myc:
<400> 10
   gctagctagc gccaccatgg aacaaaaact catctcagaa gaggatctgc ctgaggaaac 60
   ccagacccaa gac 73
<210> 11
   <211> 91
   <212> DNA
   <213> Artificial
<220>
   <223> Hsp90¦Á-pc3.1-Nhe1-For-His-Myc:
<400> 11
   gctagctagc gccaccatgc atcatcatca tcatcatgaa caaaaactca tctcagaaga 60
   ggatctgcct gaggaaaccc agacccaaga c 91
<210> 12
   <211> 34
   <212> DNA
   <213> Artificial
<220>
   <223> Hsp90¦Á-pc3.1-Xho1-Re-nostop
<400> 12
   cccgctcgag tgtctacttc ttccatgcgt gatg 34
<210> 13
   <211> 37
   <212> DNA
   <213> Artificial
<220>
   <223> Hsp90¦Á-EE-AA
<400> 13
   ggccgctcga gtgtctactg ctgccatgcg tgatgtg 37
<210> 14
   <211> 37
   <212> DNA
   <213> Artificial
<220>
   <223> Hsp90¦Á-VD-AA
<400> 14
   ggccgctcga gttgctgctt cttccatgcg tgatgtg 37
<210> 15
   <211> 37
   <212> DNA
   <213> Artificial
<220>
   <223> Hsp90¦Á-EEVD-AAAA
<400> 15
   ggccgctcga gttgctgctg ctgccatgcg tgatgtg 37
<210> 16
   <211> 33
   <212> DNA
   <213> Artificial
<220>
   <223> Hsp90¦Á-C¦¤4-Xho
<400> 16
   ccgctcgagt catgcgtgat gtgtcgtcat ctc 33
<210> 17
   <211> 39
   <212> DNA
   <213> Artificial
<220>
   <223> Hsp90¦Á-T89A-Sense
<400> 17
   gatcgaactc ttgcaattgt ggatactgga attggaatg 39
<210> 18
   <211> 39
   <212> DNA
   <213> Artificial
<220>
   <223> Hsp90¦Á-T89-AntiSense
<400> 18
   cattccaatt ccagtatcca caattgcaag agttcgatc 39
<210> 19
   <211> 39
   <212> DNA
   <213> Artificial
<220>
   <223> PP5-NheI-For
<400> 19
   ctagctagca tgtacccata cgacgtccca gactacgct 39
<210> 20
   <211> 40
   <212> DNA
   <213> Artificial
<220>
   <223> PP5-XhoI-Re
<400> 20
   ccgctcgagt taatgatgat gatgatgatg cacgtgtacc 40